(19) 

**Europäisches Patentamt**
**European Patent Office**
**Office européen des brevets**

(11) **EP 3 287 530 A1**

(12) **EUROPEAN PATENT APPLICATION**
published in accordance with Art. 153(4) EPC

(43) Date of publication:
**28.02.2018 Bulletin 2018/09**

(21) Application number: **16783407.6**

(22) Date of filing: **20.04.2016**

(51) Int Cl.:
*C12Q 1/68* (2018.01)          *B01L 7/00* (2006.01)
*G06F 19/00* (2018.01)

(86) International application number:
**PCT/KR2016/004130**

(87) International publication number:
**WO 2016/171474 (27.10.2016 Gazette 2016/43)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **20.04.2015 KR 20150055227**

(71) Applicant: **KIM, Sung-Chun**
**Seoul 03733 (KR)**

(72) Inventor: **KIM, Sung-Chun**
**Seoul 03733 (KR)**

(74) Representative: **Betten & Resch**
**Patent- und Rechtsanwälte PartGmbB**
**Maximiliansplatz 14**
**80333 München (DE)**

(54) **METHOD FOR ANALYZING BIOMOLECULE BY USING EXTERNAL BIOMOLECULE AS STANDARD MATERIAL, AND KIT THEREFOR**

(57)      This invention generally relates to a method of analyzing biomolecules, and particularly to a ligand-nucleic-acid analysis method, kit, apparatus, and system for analyzing one or more biomolecules according to a nucleic acid analysis technique using a standard material for quality control and measurement in order to provide clinical decision support with respect to the biomolecules, and a clinical decision support system using the same.

**Description**

**Technical Field**

The present invention relates to a method and a kit for analyzing a biomolecule using nucleic acid analysis.

**Background Art**

Biomolecules include many materials such as proteins, nucleic acids, lipids, and carbohydrates, which constitute biosamples. A technique for analyzing the biomolecules and a technique for generating information that quantitative states of the biomolecules in the biosamples are integrated, that is, information on multiple tests of the biomolecules, have been extensively developed with the development of physics, biochemistry, and bioinformatics. However, conventional methods or devices have problems with regard to the use, maintenance costs, ease of use, accuracy, sensitivity, test time, and process automation thereof. Accordingly, there is strong demand for novel effective methods and devices.

Biomolecule analysis and multiple test techniques are not the ultimate goal but a means for achieving the goal. The techniques may be useful for microorganisms, cells, and tissues, thus being extensively applied to medicine, veterinary medicine, environmental engineering, food engineering, and agriculture.

Analysis and profiling of biomolecules including proteins and organic materials constituting tissues, cell masses, cells, and microorganisms, which are biosamples, are performed by various methods using the physical and chemical properties thereof.

A clinical decision support system for analyzing biomolecules in a biosample and for analyzing the biological meaning using the analysis result is a system for supporting decision making on diagnosis and treatment by a doctor during patient treatment. The clinical decision support system is broadly divided into a case-based machine-learning inference system and an expert system. The case-based machine-learning inference system is a system that collects clinical information and biological information, that is, data resulting from biomolecule analysis, of patients who have been diagnosed with a disease, and then infers or discriminates diseases based on the given clinical information and biological information using machine learning. The expert system is a system that diagnoses diseases using rules set by medical experts in advance.

Recently, a protein chip or an aptamer chip (Smith et al., Mol Cell Protomics , 11-18. 2003 and McCauley et al., Anal Biochem, 319 (2), 244-250. 2003) has been developed and used as a high-throughput screening technique for a protein body, and is a representative technique for performing multiple tests on biomolecules. Examples of supports used for high-throughput screening include glass slides, sensing surfaces of biosensors, beads, and nanoparticles.

Further, a method of performing multiple test analysis to determine useful biomolecules and separating the biomolecules and then identifying constituent components thereof using MALDI-TOF (Matrix assisted laser desorption/Ionization-time of flight) has been performed. Recently, much research has been conducted on protein profiling using SELDI-TOFMS (Surface-enhanced laser desorption/ionization time of flight mass spectrometry) (Adam et al., Cancer Research, 62, 3609-3614. 2002; Li et Al., Clinical Chemistry, 48, 1296-1304. 2002; and Petricoin et al., The Lancet, 359, 572-577, 2002). Another approach has been to develop an immuno-PCR (IPCR) method, which is a technique for amplifying signals using DNA and nucleic acid polymerase (Sano et al., Science 258, 120-122, 1992).

As described above, the method of analyzing the biomolecules has been developed to improve detection sensitivity and multiple analysis capability. However, the analysis method faces technological challenges pertaining to standard materials for quality control and measurement, reduction in analysis costs, shortening of the analysis time, an improvement in sensitivity, and an increase in reproducibility.

**Disclosure**

**Technical Problem**

As a result of studying to overcome the problems of conventional techniques for analyzing biomolecules and to develop a technique for performing real-time analysis of the biomolecules with improved efficiency and sensitivity, a method of quantitatively analyzing one or more biomolecules using external biomolecules as a standard material according to nucleic acid analysis in a single test has been developed, thereby achieving the object of the present invention.

**Technical Solution**

In order to accomplish the above object, an aspect of the present invention provides a method of analyzing biomolecules using nucleic acid analysis.

The method of analyzing the biomolecules using the nucleic acid analysis according to the present invention includes (a) preparing a biosample containing one or more target biomolecules or a processed sample thereof, (b) adding a connection structure of a ligand and a design nucleic acid specifically binding to the target biomolecules to thus form complexes of the biomolecules and the specific connection structure thereof, (c) separating the complexes, (d) amplifying the design nucleic acid of the complexes, and (e) obtaining qualitative or quantitative information on the target biomolecules by analyzing an amplified product thereof. The design nucleic acid includes a region to be recognized and bound by a forward primer, a region to be recognized and bound by a backward primer, and between the regions, a region representing molecules specifically binding to the ligand so that the molecules are capable of being identified.

Since the design nucleic acid includes the region representing the molecules specifically binding to the ligand connected thereto, whether or not the amplified substance of the design nucleic acid is present and/or the amount of the amplified substance that is present (i.e., the amplification amount) may be decided (precisely, whether or not the amplified substance of the region representing the molecules specifically binding to the ligand is present and/or the amount of the amplified substance that is present (i.e., amplification amount) may be decided), whereby the molecules specifically binding to the ligand connected to the design nucleic acid are analyzed qualitatively (i.e., whether or not the molecules are present is confirmed) or quantitatively (i.e., the amount of the molecules that are present is checked in relative or absolute terms).

The region representing the molecules specifically binding to the ligand refers to a region designed depending on the molecule specifically binding to the ligand. Any design may be used as long as it enables detection of a difference, but it will usually be done with different base sequences or different lengths.

In the method of the present invention, even when two or more biomolecules to be analyzed are present, the connection structure of the ligand and the design nucleic acid specifically binding to each of the target biomolecules is added, and one set of the forward primer and the backward primer is used to perform amplification by a multiplex polymerase chain reaction (real-time multiplex PCR), whereby the two or more target biomolecules may be simultaneously analyzed qualitatively and/or quantitatively in a single amplification process (i.e., in a single test or in a single tube). Even when two or more the ligands to which the design nucleic acid is coupled are different each other and thus biomolecules to be recognized by the ligands are also different each other, since designing is performed so that the region complementary to the forward primer and the region complementary to the backward primer have a common base sequence among all connection structures of the ligands and the design nucleic acids, all of the design nucleic acids of the connection structures may be amplified using one set of the forward primer and the backward primer.

In the method of the present invention, the ligand and the design nucleic acid may be covalently or non-covalently coupled to each other, thus obtaining the connection structure of the ligand and the design nucleic acid. Any methods known in the art may be used for the coupling. For example, as shown in Examples of the present invention, an amine group may be introduced at the 5' end of the design nucleic acid to be covalently coupled to the ligand (an antibody which is a protein), or non-covalent coupling may be performed using biotin and avidin as media (biotin is bonded to the ligand and avidin is bonded to the design nucleic acid).

In the method of the present invention, for example, the processed sample of the biosample refers to plasma or serum obtained by processing the biosample when the biosample is blood, or refers to a total protein sample obtained from the biosample using a total protein extraction reagent when the target biomolecule is a protein.

In the method of the present invention, a standard material may be used for relative and/or absolute quantitative analysis. An external biomolecule (external standard material) that is not present in a sample to be analyzed may be used as the standard material.

If the external standard material is a material that is not present in the sample to be analyzed, examples thereof may include a predetermined material, for example, Enoyl-ACP reductase, which is an *E. coli*-derived protein used in the Examples below, in the case where the sample to be analyzed is a human-derived biosample and the target biomolecule is a protein.

When the external biomolecule is used as the standard material, the connection structure of the ligand and the design nucleic acid specifically binding to the standard material is added together with the connection structure of the ligand and the design nucleic acid specifically binding to the target biomolecule, thus forming complexes. After all complexes that are formed (all of the complexes with one or more biomolecules and the complexes with the standard material) are separated, the design nucleic acid of the complex is amplified using one set of the forward primer and the backward primer, and the amplified substance for the external biomolecule and the amplified substance for the target biomolecule are quantified and compared, thereby relatively or absolutely quantifying the biomolecule. When the external biomolecule is used as the standard material, the external biomolecule needs to be quantified and added in advance (before formation of the complexes) to the biosample to be analyzed or the processed sample thereof. When the external biomolecule that is quantified is used as the standard material, the standard material may be subjected to analysis that is different from the analysis of the biomolecule, thus absolutely quantifying the target biomolecule without preparing a calibration curve.

In the case where the external biomolecule is used as the standard material, when there is a difference between the binding affinity of the target biomolecule and the connection structure thereof and the binding affinity of the external biomolecule and the connection structure thereof, the quantification result may not accurately reflect the amount (or concentration) of the target biomolecule in the sample.

Therefore, when the external biomolecule is used as the standard material, it is preferable to perform calibration of the quantification result reflecting the difference between the binding affinity of the target biomolecule and the connection structure thereof and the binding affinity of the external biomolecule and the connection structure thereof.

The reflection of the difference may include (i) quantifying the target biomolecules and the external biomolecules to thus prepare a control sample including the target biomolecules and the external biomolecules at the same concentration, (ii) adding the connection structure which is specifically bound to the target biomolecules or the external biomolecules of the control sample and which is identical with the connection structure used in the analysis method of the present invention to thus form a complex of the target biomolecules and the specific connection structure thereof and a complex of the external biomolecules and the specific connection structure thereof, (iii) separating the complexes, (iv) amplifying design nucleic acids of the complexes, (v) obtaining a difference between the amounts of an amplified substance for the target biomolecules and an amplified substance for the external biomolecules, and (vi) reflecting the difference to the quantification result of the target biomolecules.

The target biomolecule is not particularly limited as long as the target biomolecule can form a ligand which recognizes proteins, peptides, polysaccharides, lipids, or molecules formed by bonding two or more thereof (glycoproteins, glycolipids, lipid proteins, or lipid polysaccharides), and which is bonded thereto.

Further, the design nucleic acid may be a double-stranded nucleic acid (DNA-DNA, DNA-RNA, or RNA-RNA) or a single-stranded nucleic acid (DNA or RNA). As in the Examples below, the design nucleic acid may be a nucleic acid which is chemically modified, for example, which includes an amine group introduced at a 5' end thereof for the purpose of ease of coupling with an antibody which is a ligand.

In the method of the present invention, the amplified product may be analyzed using a titration detection material such as a Taqman probe during amplification when the amplification includes a real-time polymerase chain reaction (real-time PCR), a real-time reverse-transcription-polymerase chain reaction (real-time RT-PCR), a real-time multiplex polymerase chain reaction (real-time multiplex PCR), or a real-time multiplex reverse-transcription-polymerase chain reaction (real-time multiplex RT-PCR). Further, the analysis may be performed using a micro-array to which a probe (usually a single-stranded oligonucleotide having a base sequence complementary to the amplified substance) for capturing the amplified substance of the design nucleic acid (specifically, the amplified substance of the region representing the molecules) is fixed, and may be performed using capillary electrophoresis as in the Examples below. This analysis of the amplified product may provide qualitative and/or quantitative information on the target biomolecule.

Another aspect provides a kit for analyzing biomolecules using external biomolecules as a standard material.

The analytical kit may include one or more connection structures of a ligand and a design nucleic acid specifically binding to target biomolecules.

In a preferred aspect, the analytical kit includes one or more types of connection structures of a ligand and a design nucleic acid specifically binding to each of one or more types of target biomolecules, and also one or more types of connection structures of the ligand and the design nucleic acid specifically binding to each of one or more types of the external biomolecules, which are not present in a biosample containing the target biomolecules, as the standard material.

As described above, the design nucleic acid includes a region to be bound by a forward primer, a region to be bound by a backward primer, and between the regions, a region representing molecules specifically binding to the ligand so that the molecules are capable of being identified. The region of the forward primer and the region of the backward primer in the design nucleic acid have a common base sequence in all of the design nucleic acids so that all of the design nucleic acids are amplified using one set of the forward primer and the backward primer.

In the preferred aspect, the analytical kit may further include one or more types of the target biomolecules and one or more types of the external biomolecules to be used in a control sample in order to determine a difference between the binding affinity of the target biomolecules and the connection structures thereof and the binding affinity of the external biomolecules, which are the standard material, and the connection structures thereof.

In another preferred aspect, the kit may further include an instruction manual thereof, and the instruction manual may instruct the above-described analysis method of the present invention as a method of analyzing biomolecules according to the protocol.

In another preferred aspect, the manual included in the kit may further teach that the quantification information on the target biomolecules reflects a difference between the binding affinity of the target biomolecules and the connection structure thereof and the binding affinity of the external biomolecules and the connection structure thereof..

In another preferred aspect, the manual included in the kit may further teach that the reflecting of the difference between the binding affinities is performed according to the above-described method.

Hereinafter, the present invention will be described in detail.

Biomolecules may be proteins, peptides, nucleic acids, carbohydrates, lipids, polysaccharides, glycoproteins, hormones, acceptors, antigens, antibodies, viruses, pathogens, toxic materials, substrates, metabolites, transition state analogs, cofactors, inhibitors, drugs, dyes, nutrients, growth factors, cells, or tissues, but are not limited thereto. The biomolecules may be any chemical or biological effectors, and mean target molecules that may have any size.

The biomolecules may be analyzed in a biosample, thus checking a change in the body and objectively measuring the normal or pathological condition of the living organism and the extent of reaction to the drug, which leads to the determination of biological meaning.

A ligand is a material that binds to the biomolecules, and representative examples thereof include an antibody, an aptamer, and a peptide. The antibody is a material specifically binding to the biomolecules, and is a protein that specifically binds to an epitope of the antigen, causing an antigen-antibody reaction. The aptamer means small (20 to 60 nucleotides) single-stranded nucleic acid (DNA or RNA) fragments which have high affinity to various receptors ranging from low-molecular weight compounds to proteins and which specifically bind to the receptors.

A standard material is a material for quality control and measurement, and may be an internal material that is always present in a predetermined amount in a sample to be analyzed, or may be external biomolecules which bind to a non-reactive ligand together with the biomolecules constituting the sample by a biological analysis method.

Preferably, in the case of typical biomolecule analysis, internal quality control is performed using biomolecules contained in a biosample to be analyzed for comparison during various examinations and tests. The ideal material for quality control is a material which is always present in the sample to be analyzed in a predetermined amount, but if not, external materials which are not contained in the sample may be used. Preferably, when the sample to be analyzed is the biosample, the material for quality control may include external biomolecules which are not contained in the biosample. "Quality control" means internal quality control in which the accuracy of measurement values is controlled by analyzing a group of test results obtained from each measurement without using external standard materials such as samples for control.

Preferably, in the present invention, when a human-derived biosample is analyzed using a standard material for quality control and measurement, plant-specific biomolecules may be used as the biomolecules which are not included in the human-derived biosample. Genomic projects of humans, *E. coli,* and *Arabidopsis thaliana* which is a species of plant, have now been completed, and species-specific proteins have been reported. In the present invention, a protein that is specific to *E. coli* or a plant may be used as a standard material in order to analyze a human-derived biosample.

The method of measuring and evaluating the concentration of biomolecules should be reliable in terms of accuracy, precision, and reproducibility. The reason for the importance of precision in analyzing biomolecules is that the samples have very complicated compositions such as various types of materials including proteins and that the target biomolecules to be analyzed are present in small amounts. Accordingly, there is very high demand for the standard material for quality control and measurement in order to analyze a small amount of components.

Inter-laboratory variation in analytical values may occur due to the effect of the materials, which are present in the sample and disturb the analysis, and due to the analysis of small amounts. In order to reduce such inter-laboratory variation, a standard material for quality control and measurement, which homogeneously contains the biomolecules at the same concentration, is needed for the samples to be analyzed. The analytical values obtained from various laboratories are compared using a standard material to determine the state of analysis instruments, which leads to accurate analysis conditions. Further, there is a need for a biomolecule analysis method in which a standard material is homogeneously distributed in a sample to be analyzed, thereby minimizing variation between samples and improving the reliability of analysis.

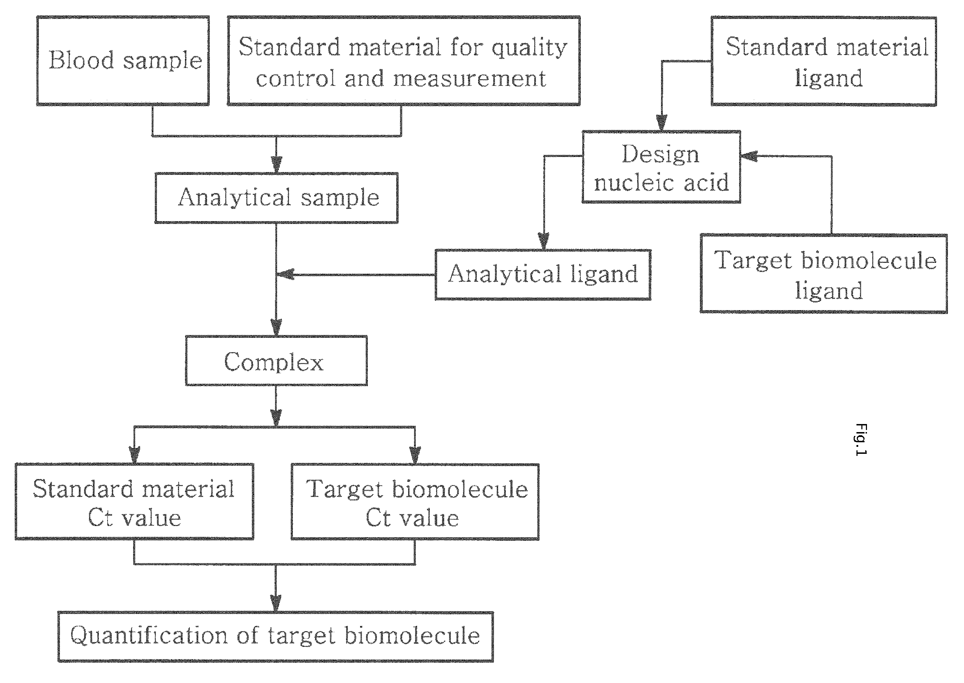

Therefore, the present invention provides a tentative ligand-nucleic-acid analysis method, which is a technique for analyzing target biomolecules and a standard material for quality control and measurement together using a nucleic acid analysis technique, as the concept shown in FIG. 1, thereby overcoming test limits pertaining to analysis of the biomolecules, accuracy, precision, and reproducibility.

In the present invention, in order to analyze one or more biomolecules, the standard material for quality control and measurement is an internal material that is always present in a predetermined amount in a sample to be analyzed, or is external biomolecules which bind to a ligand having no binding reactivity together with the biomolecules constituting the sample by the ligand-nucleic-acid analysis method.

A ligand-nucleic-acid analysis method, kit, and apparatus are provided, and the analysis is performed using the base sequence and the length of design nucleic acids representing the standard material and the target nucleic acid molecules.

The design nucleic acid is a nucleic acid including nucleotides so that analysis is feasible, and represents the standard material and the specific biomolecules to be analyzed (tentative target biomolecules) by the base sequence or the length of the base sequence. The design nucleic acid is bonded to the ligand that binds to the standard material and the target biomolecules, and the signal indicating the presence of the standard material and the biomolecules is amplified using a nucleic acid amplification method, thereby analyzing the amplified product.

In the case of the design nucleic acid representing the standard material and the target biomolecules, there must be no reactivity of hybridization, which is a kind of biological analysis method, between the design nucleic acids representing the standard material and the target biomolecules, the lengths of the design nucleic acids must be different from each other, and the design nucleic acids must be analyzable using the nucleic acid analysis technique. Preferably, a pair of PCR primers used for amplification of the design nucleic acid may be used so that the analysis is performed using a method which is the same as a method of analyzing the design nucleic acid.

A structure in which the design nucleic acid is connected to the ligand corresponding to a specific biomolecule is referred to as an analytical ligand, and a structure in which the design nucleic acid is connected to the ligand corresponding to a standard material is referred to as a standard analytical ligand.

An "amplification reaction (polymerase chain reaction)" or "PCR", which is a method of amplifying the signal indicating the presence of the design nucleic acid in order to analyze the design nucleic acid, means a reaction for amplifying target nucleic acid molecules. A variety of amplification reactions have been reported in the art, including a polymerase chain reaction (PCR) (U.S. Pat. Nos. 4,683,195, 4,683,202, and 4,800,159), a method of a reverse-transcription polymerase chain reaction (RT-PCR) (Sambrook et al., Molecular Cloning. A Laboratory Manual, 3rd ed. Cold Spring Harbor Press (2001)), a multiplex PCR (McPherson and Moller, 2000), a ligase chain reaction (LCR) (Stemmer, W.P., et al., 1995, Gene, 164, 49-53; Carlson, B., 2008, Genet Eng Biotechn N, 28, 12-12), a Gap-LCR (WO 90/01069), a repair chain reaction (EP 439,182), transcription-mediated amplification (TMA) (SantaLucia, J., 1998, Proc Natl Acad Sci U S A, 95, 1460-1465), self-sustained sequence replication, selective amplification of target polynucleotide sequences (U.S. Pat. No. 6,410,276), a consensus-sequence-primed polymerase chain reaction (CPPCR) (U.S. Pat. No. 4,437,975), an arbitrarily primed polymerase chain reaction (AP-PCR) (U.S. Pat. Nos. 5,413,909 and 5,861,245), nucleic acid sequence based amplification (NASBA) (U.S. Pat. Nos. 5,130,238, 5,409,818, 5,554,517, and 6,063,603), and strand displacement amplification, but are not limited thereto.

In the present invention, a design nucleic acid that is designed to increase the test limit of the target biomolecules is used. The signal indicating the presence thereof is firstly amplified using a nucleic acid amplification technique and is then amplified using a fluorescent material. Amplification of the nucleic acid using the PCR may serve to increase resolution.

Preferably, the constitution of the design nucleic acid connected to the specific ligand in analytical ligand and standard analytical ligand structures in order to implement the PCR method of amplifying the present signal of the design nucleic acid is represented by the following General Formula (I).

$$5\text{'-P1-N}_v\text{-P3-3'} \qquad (I)$$

In General Formula (I), the P1 region and the P3 region are regions complementary to a forward primer and a backward primer, respectively, and the Nv region is a portion that represents a ligand binding to a specific biomolecule by a specific base sequence or a length thereof (i.e., a region representing the specific biomolecule) and facilitates the qualitative and quantitative analysis of the specific biomolecule. This Nv region preferably has a length of 10 to 200 nucleotides.

Further, in order to facilitate coupling to the ligand, preferably, an amine group, which is a reactive group, may be present at the 5' end, and a spacer including about 10 nucleotides may be present upstream of the region binding to the forward primer.

Preferably, a pair of universal primers may be used as the forward primer and the backward primer.

In the pair of PCR primers, the forward primer may include a base sequence that completely complementarily binds to the base sequence of the design nucleic acid, and the backward primer may be identical to the base sequence of the design nucleic acid. Preferably, the PCR primers may be a pair of universal PCR primers.

The typical universal PCR primers are oligonucleotides, which are base sequences frequently used for sequencing or PCR and are widely used in commercial cloning vectors. The universal PCR primers mean oligonucleotides ranging from 14 mer to 40 mer. The universal PCR primers may act as a beginning point of synthesis under a condition in which the synthesis of a primer extension product complementary to a template is induced, that is, under the condition of the presence of polymerases such as nucleotides and DNA polymerases and appropriate temperature and pH conditions.

The "amplified product" means the product of a polynucleotide amplification reaction. That is, the amplified product is generally a group of polynucleotides that are double-stranded and replicated from one or more starting sequences. The one or more starting sequences may be one or more copies of the same sequence, or may be a mixture of different sequences. The amplified product may be generated according to a variety of amplification reactions, and the products of the amplification reactions are multiple copies of one or more target nucleic acids. In the present invention, the amplified product of the design nucleic acid may be analyzed using a nucleic acid analysis technique, thereby analyzing the target biomolecules in the sample.

According to the present invention, there is provided a method for representing at least one target biomolecule of the design nucleic acid. The method includes bonding a reactive group N in the P1 portion of the design nucleic acid to a reactive group N of a ligand, and at least one target biomolecule of the design nucleic acid is represented by the bonding.

The design nucleic acid and the ligand may be connected in various ways, and preferably, the reactive group is bonded to each of the design nucleic acid and the ligand, which manufactures the analytical ligand due to a chemical reaction. According to the present invention, analytical ligands are provided. In the analytical ligand according to the present invention, one or more design nucleic acids are bonded to the reactive group N located in the ligand.

Preferably, the bonding of the design nucleic acid facilitates representation of the target biomolecules and analysis using the nucleic acid analysis technique or amplification of the signal indicating the presence thereof.

Preferably, the reactive group N includes one or more groups selected from among an amino group, a carboxyl group, a thiol group, alkyl, alkenyl, alkynyl, oxoalkyl, oxoalkenyl, oxoalkynyl, aminoalkyl, aminoalkenyl, aminoalkynyl, sulfoalkyl, sulfoalkenyl, a sulfoalkynyl group, phosphoalkyl, phosphoalkenyl, and phosphoalkynyl.

According to the present invention, there is provided a design nucleic acid used for analyzing a disease or a condition in an individual having cells, tissue, or fluid containing target biomolecules, and the design nucleic acid efficiently represents the ligand that specifically binds to the target biomolecules.

Methods for harvesting the complex of the biomolecules and the ligand may be broadly classified into two types. One method is a method of nonspecifically binding the biomolecules of a sample to be analyzed to a solid support. Preferably, the solid support is a plate or a nitrocellulose membrane used in an ELISA (enzyme-linked immunosorbent assay) method. Another method is a method of binding the ligands of the standard material and the target biomolecule to a solid support, and the solid support is preferably a magnetic bead.

In the present invention, a structure used to harvest the complex of the target biomolecules and the ligand is referred to as a harvest ligand, and a structure used to harvest the complex of the standard material and the ligand is referred to as a standard harvest ligand.

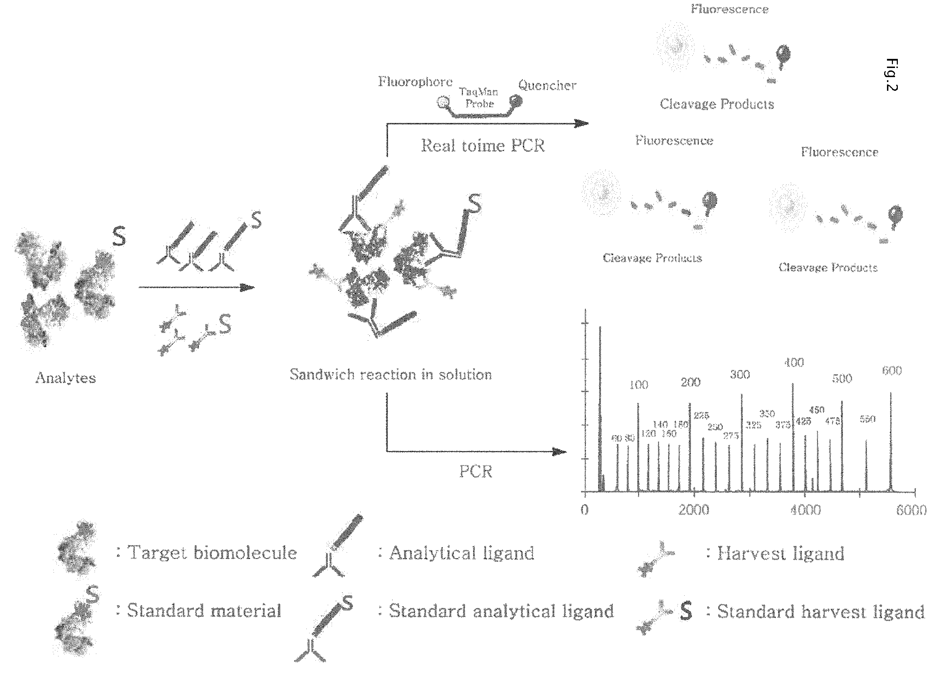

As shown in FIG. 2, in order to analyze one or more target biomolecules in a sample which contains a standard material having a known concentration and which is to be analyzed, the base sequence or the length of the design nucleic acids of the standard material and the target biomolecule may be analyzed using the amplified product obtained by amplifying the design nucleic acid according to a nucleic acid analysis technique, thereby analyzing the target biomolecules. Preferably, the result of analysis of the target biomolecule includes an amount or an amount ratio of the target biomolecules that are present, or a ratio of the standard material and the target biomolecules.

The base sequence of the design nucleic acid may be determined depending on the target biomolecules and the standard material to thus perform representing, thereby analyzing the corresponding target biomolecules. Preferably, qualitative and quantitative analyses of one or more target biomolecules may be simultaneously performed using the base sequences of the amplified products of the design nucleic acids corresponding to the target biomolecules and the standard material.

Preferably, after the base sequence of the amplified product is determined using a bead array or a microarray according to a method using a FRET probe based on hybridization and real-time PCR, or a method using a capture probe and hybridization, whether or not the target biomolecules are present and the amount of the target biomolecules that are present may be analyzed.

After one or more types of design nucleic acids are amplified, amplicon detection may be laborious and time-consuming. There are real-time methods to monitor amplification during the PCR process. Typical examples of the ideal methods used to analyze a particular type of nucleic acid include fluorescently labeled probes that are annealed to newly synthesized DNA.

In order to simultaneously analyze the standard material and one or more target biomolecules in a sample containing a target material having a known concentration in one tube, design nucleic acids which correspond thereto and which have different base sequences are manufactured. The design nucleic acids are coupled to ligands to be bound to the target biomolecules, thus preparing analytical ligands. The analytical ligands are reacted with samples to be analyzed to harvest an analytical-ligand-biomolecule-harvest-ligand complex and a standard-analytical-ligand-standard-material-standard-harvest-ligand complex. The design nucleic acids of the complexes that are harvested may be subjected to PCR using a pair of universal PCR primers as a template, thereby synthesizing amplified products.

In order to analyze the ligands and the design nucleic acids of one or more target biomolecules containing the standard material, a FRET probe including a probe complementarily binding to the base sequence of the design nucleic acid may be manufactured.

The FRET probe is generally designed such that donor emission is quenched in the absence of the target due to FRET (fluorescence resonance energy transfer) between two chromophores. When a donor chromophore and an acceptor chromophore in a pair thereof are located adjacent to each other, the donor chromophore in an excited state may transfer energy to the acceptor chromophore. This transfer is always non-radiative and may occur due to dipole-dipole coupling. When the distance between the chromophores is sufficiently increased, the FRET efficiency is lowered and the donor chromophore emission may be radially detected. Examples of the donor chromophores include FAM (6-carboxyfluorescein), TAMRA, VIC, JOE, Cy3, Cy5, and Texas Red. The excitation spectrum of the acceptor chromophore is selected so as to overlap the emission spectrum of the donor chromophore. An example of such a pair is FAM-TAMRA. Further, there is a non-fluorescent acceptor that quenches a wide range of donors. Other examples of donor-acceptor FRET pairs are known in the art.

Typical examples of the FRET probes that may be used for real-time detection of the PCR include molecular beacons (e.g., U.S. Pat. No. 5,925,517), TaqMan probes (e.g., U.S. Pat. Nos. 5,210,015 and 5,487,972), and CATACLEAVE probes (e.g., U.S. Pat. No. 5,763,181).

The molecular beacon is a single-stranded oligonucleotide designed so that the probe forms a secondary structure in which the donor chromophore and the acceptor chromophore are adjacent to each other in a non-binding state and donor emission is reduced. At an appropriate reaction temperature, the beacon is unfolded and specifically binds to the amplicon. Once the beacon is unfolded, the distance between the donor chromophore and the acceptor chromophore may increase, FRET may be reversed, and donor emission may be monitored using specialized apparatuses. The TaqMan and CATACLEAVE techniques are different from the molecular beacon in that the FRET probe that is used is cleaved, which sufficiently separates the donor chromophore and the acceptor chromophore so as to reverse the FRET.

In the Taqman technique, a single-stranded oligonucleotide probe which is labeled with the donor chromophore at a 5' end and with the acceptor chromophore at a 3' end is used. The DNA polymerase used for amplification should have 5'3' exonuclease activity. The TaqMan probe binds to the primer and also to one strand of the amplicon. As the DNA polymerase extends the primer, the polymerase may ultimately meet with the bound TaqMan probe. The exonuclease activity of the polymerase may start at the 5' end to sequentially degrade the TaqMan probe. As the probe is digested, the mononucleotide containing the probe is released into a reaction buffer. The donor is diffused away from the acceptor and FRET is reversed. The emission from the donor is monitored in order to confirm the probe cleavage. Because of the way in which Taqman works, a specific amplicon may be detected only once per cycle of the PCR.

The extension of the primer through a TaqMan target site generates a double-stranded product that prevents further binding of the TaqMan probe until the amplicon is denatured in the next PCR cycle.

U.S. Pat. No. 5,763,181, the contents of which are incorporated herein by reference, describes another real-time detection method (referred to as "CATACLEAVE"). The CATACLEAVE technique is different from the TaqMan in that the cleavage of the probe is accomplished by a second enzyme that does not have polymerase activity. The CATACLEAVE probe has a sequence within the molecule that is the target of an endonuclease, for example, a restriction enzyme or an RNase. In one example, the CATACLEAVE probe has a chimeric structure in which the 5' and 3' ends of the probe include DNAs and the cleavage site includes RNAs.

In another Example of the present invention, the Taqman probe is labeled with a fluorescent reporter dye at a 5' end thereof, and has a quencher at a 3' end thereof, and has a sequence complementarily binding to the design nucleic acid between the reporter and quencher.

The reporter molecules and the quencher molecules to be coupled to the TaqMan probe include a fluorescent material and a non-fluorescent material.

The fluorescent reporter molecules and quencher molecules that may be used in the present invention may be any of those known in the art, examples of which are as follows (the numbers in parentheses are maximum emission wavelengths in nanometers): Cy2™ (506), YOPRO™-1 (509), YOYO™-1 (509), Calcein (517), FITC (518), FluorX™ (519), Alexa™ (520), Rhodamine 110 (520), 5-FAM (522), Oregon Green™ 500 (522), Oregon Green™488 (524), RiboGreen™ (525), Rhodamine Green™ (527), Rhodamine 123 (529), Magnesium Green™ (531), Calcium Green™ (533), TO-PROTM-1 (533), TOTO1 (533), JOE (548), BODIPY530/550 (550), Dil (565), BODIPY TMR (568), BODIPY558/568 (568), BODIPY564/570 (570), Cy3™ (570), Alexa™546 (570), TRITC (572), Magnesium Orange™ (575), Phycoerythrin R&B (575), Rhodamine Phalloidin (575), Calcium Orange™ (576), Pyronin Y (580), Rhodamine B (580), TAMRA (582), Rhodamine Red™ (590), Cy3.5™ (596), ROX (608), Calcium Crimson™ (615), Alexa™594 (615), Texas Red (615), Nile Red (628), YO-PRO™-3 (631), YOYOTM-3 (631), R-phycocyanin (642), CPhycocyanin (648), TO-PROTM-3 (660), TOTO3 (660), DiD DilC(5) (665), Cy5™ (670), Thiadicarbocyanine (671), Cy5.5 (694), HEX (556), TET (536), VIC (546), BHQ-1 (534), BHQ-2 (579), BHQ-3 (672), Biosearch Blue (447), CAL Fluor Gold 540 (544), CAL Fluor Orange 560 (559), CAL Fluor Red 590 (591), CAL Fluor Red 610 (610), CAL Fluor Red 635 (637), FAM (520), Fluorescein (520), Fluorescein-C3 (520), Pulsar 650 (566), Quasar 570 (667), Quasar 670 (705), and Quasar 705 (610). The numbers in parentheses are maximum emission wavelengths in nanometers. According to an embodiment of the present invention, the reporter molecules and the quencher molecules include Cy5, ROX, HEX, FAM, BHQ-1, BHQ-2, or Cy5.5-based labels.

Suitable reporter-quencher pairs are disclosed in many documents: Pesce et al., editors, FLUORESCENCE SPECTROSCOPY (Marcel Dekker, New York, 1971); White et al., FLUORESCENCE ANALYSIS: A PRACTICAL APPROACH (Marcel Dekker, New York, 1970); Berlman, HANDBOOK OF FLUORESCENCE SPECTRA OF AROMATIC MOLECULES, 2nd EDITION (Academic Press, New York, 1971); Griffiths, COLOUR AND CONSTITUTION OF ORGANIC MOLECULES (Academic Press, New York, 1976); Bishop, editor, INDICATORS (Pergamon Press, Oxford, 1972); Haugland, HANDBOOK OF FLUORESCENT PROBES AND RESEARCH CHEMICALS (Molecular Probes, Eugene, 1992); Pringsheim, FLUORESCENCE AND PHOSPHORESCENCE (Interscience Publishers, New York, 1949); Haugland, R.P., HANDBOOK OF FLUORESCENT PROBES AND RESEARCH CHEMICALS, Sixth Edition, Molecular Probes, Eugene, Oreg., 1996; U.S. Pat. Nos. 3,996,345 and 4,351,760.

According to embodiments of the present invention, the 5'-end of the probe of the present invention is labeled with one type of fluorophore selected from the group consisting of Cy5, ROX, HEX, FAM, and Cy5.5, and the 3'-end thereof may be modified using one type of quencher selected from the group consisting of BHQ-1 and BHQ-2, without being limited thereto.

The present invention provides a ligand-nucleic-acid analysis method, kit, and apparatus for analyzing five or more types of target biomolecules including the standard material using the FRET probe.

TOCE (Tagging oligonucleotide cleavage and extension) is a technique for detecting multiple (five or more) target genetic materials and for checking genetic mutation thereof (Lee, D.H. TOCE: Innovative Technology for High Multiplex Real-time PCR. Seegene bulletin 1, 510 (2012)) .

In the present invention, in order to analyze one or more biomolecules in an analytical sample having a known concentration, a manufacturing method for implementing a DPO primer technique and a pitcher & catcher technique on the design nucleic acid is provided.

In the design nucleic acid structure [General Formula I], the P1 region constitutes part or all of a region complementarily binding to a DPO forward primer, and the P3 region constitutes part or all of the same base sequence as a DPO backward primer. The $N_v$ region constitutes part or all of a region complementarily binding to a pitcher primer having a tagging portion.

The present invention preferably provides a catcher oligomer including part or all of a portion to which the tagging portion complementarily binds and a template portion constituting the FRET probe for generating a signal.

The present invention provides a ligand-nucleic-acid analysis method, kit, and apparatus for releasing the tagging portion of the pitcher primer so that the tagging portion complementarily binds to the catcher oligomer to elongate and to generate the signal during amplification of the design nucleic acid using the DPO forward primer and the DPO backward primer.

The TOCE technique allows simultaneous identification of a plurality of nucleic acids in one channel. A fluorescent signal may be checked in real time, and the presence of the target may be confirmed by analyzing the melting temperature (catcher-Tm) of a catcher. The catcher, which is an artificial template having fluorescent molecules attached thereto, generates the fluorescent signal corresponding to each target. The catcher-Tm value may be adjusted by changing the length of the catcher or the base sequence thereof. For adaptation of the TOCE reaction, the catcher-Tm value may be easily changed regardless of the base sequence of the target.

In probe-type Tm analysis methods that are currently used, the target nucleic acid DNA is directly bonded, but in the TOCE method, since the fluorescent signal is generated using the catcher, the Tm value may be freely controlled by controlling changes in the length or the base sequence of the catcher. Further, in the conventional methods, there is a difference between Tm values when the base sequence of the nucleic acid is changed. However, in the TOCE method, since the base sequence is not changed, the constant Tm value is maintained.

This unique pitcher & catcher technique allows a plurality of nucleic acid tests (multiplexing) in a single channel to be performed accurately. During the real-time PCR, a cyclic-CMTA point may be set so that the presence of the nucleic acid and the quantitative analysis result thereof are confirmed simultaneously. The cyclic-CMTA point may be set at 30, 40, and 50 cycles during the real-time PCR process, and quantitative analysis may be performed depending on the shape of the melting peak according to the amount of the nucleic acid that is present in the result analysis.

Examples of methods for measuring the amount of amplified products in the real-time PCR include absolute quantification and relative quantification. Absolute quantification is performed according to a method for forming a standard curve using a standard material having a known concentration and for calculating the concentration of a target gene to be measured using the standard curve under the assumption that the PCR efficiencies of the target gene and the standard material are the same. For more accurate quantification, the target gene and an internal control material having a known concentration are simultaneously amplified to perform quantification.

Meanwhile, in quantification of mRNAs for confirming a change in gene expression of a specific target, each standard curve is formed using a reference gene and a target gene, and the extent of amplification is obtained by calculating the concentration using the standard curve. Subsequently, relative quantification is used to calculate the ratio of the target gene to the reference gene (the concentration of the target gene/the concentration of the reference gene) in order to compensate for variation between PCRs. Relative quantification is considered to be easier, more economical, and more reliable than absolute quantification, but still involves the risk of errors due to the formation of standard curves and storage.

In the present invention, the design nucleic acid in the complex harvested by simultaneously reacting one or more target biomolecules with the standard material for quality control and measurement in one tube is analyzed using multiplex real-time PCR to obtain a Ct value. Absolute quantification and relative quantification are performed using the obtained Ct value.

In the PCR, DNA is exponentially amplified every cycle, and reaches a plateau. An amplification curve is obtained by monitoring the pattern of this amplification in real time. When the amount of the PCR amplified product reaches an amount that is detectable using fluorescence, the amplification curve begins to rise, and the signal is exponentially increased and reaches the plateau.

As an initial amount of DNA is increased, since the number of cycles, during which the amount of the amplified product reaches a detectable amount, is reduced, the amplification curve appears fast. Therefore, real-time PCR using standard samples that are subjected to stepwise dilution results in amplification curves which are lined up at the same interval in descending order of the initial amount of DNA. The threshold is set at a suitable point to calculate a Ct value (threshold cycle) at which the threshold crosses the amplification curve.

A calibration curve having a straight-line relation may be formed between the Ct value and the initial amount of the template. In the case of an unknown sample, the initial amount of the template may be obtained by calculating the Ct value and comparing the Ct value to the calibration curve, as in the case of the standard sample.

In the present invention, the structure of the amplicon, which is the design nucleic acid, is the same as that shown in General Formula (I), and the amplicon is amplified using the same PCR primer pair as that shown in General Formula (I). Accordingly, all amplification efficiencies of the design nucleic acids representing one or more target biomolecules and the standard material for quality control and measurement are the same (data are not provided).

In the present invention, the design nucleic acid in the complex harvested by performing simultaneous reaction of one or more target biomolecules with the standard material for quality control and measurement in one tube was analyzed using multiplex real-time PCR, thus obtaining a Ct value. The absolute quantification and the relative quantification were performed using the obtained Ct value.

In the present invention, the structure of the amplicon, which is the design nucleic acid, is the same as that shown in [General Formula (I)], and the amplicon is amplified using the same PCR primer pair as shown in [General Formula (I)]. Accordingly, the amplification efficiencies of the design nucleic acids representing the target biomolecule and the standard material for quality control and measurement may be the same.

In the following Example of the present invention, the amount of the design nucleic acid of the complex formed by the target biomolecules in the sample to be analyzed, to which the standard material for quality control and measurement was artificially added, was obtained by analyzing the Ct (cycle threshold) value, obtained by a $2^{-ct}$ method (comparative Ct method) (Livak KJ. Comparative Ct Method. ABI PRISM 7700 Sequence Detection System. User Bulletin No. 2. Relative Quantitation of Gene Expression. PE Applied Biosystems, 1997.).

In this Ct method, quantification may be performed without preparing a calibration curve. However, it is presupposed that the PCR amplification efficiency is almost constant for all biomolecules to be measured.

The analyzed Ct value of the design nucleic acid of the complex of the target biomolecule in the sample to be analyzed, to which the standard material is added, is normalized using the Ct value of the design nucleic acid of the standard material complex to thus secure $\Delta$Ct-n($Ct_{\text{target biomolecule}}$- $Ct_{\text{external standard material}}$), and then the concentration of the target biomolecule in the sample to be analyzed, to which the standard material is added, is calculated as follows:

Concentration of external standard material x $2^{-\Delta Ct-n}$

When the binding affinity between the external standard material and the specific ligand thereof is different from the binding affinity between the target biomolecule and the specific ligand thereof, the decided concentration of the target biomolecule may not accurately reflect the concentration of the target biomolecule in the sample. Therefore, it is preferable to perform calibration with respect to the binding affinity difference.

In order to perform this calibration, first, the target biomolecules and the target external standard material are quantified, and one or more control samples containing the target biomolecules and the target external standard material at the same concentration are prepared. The control sample(s) are used to obtain the Ct value (or the average of the values when the number of control samples is two or more) calculated from the amplified substance of the design nucleic acid of the complex of the target biomolecule and the Ct value (or the average of the values when the number of control samples is two or more) calculated from the amplified substance of the design nucleic acid of the complex of the external standard material, thus generating $\Delta$Ct-c, which is the difference between the Ct values ($Ct_{\text{target biomolecule of control sample}}$ - $Ct_{\text{external}}$ standard material of control sample) . Next, $\Delta$Ct-cal ($\Delta$Ct-n - $\Delta$Ct-c) may be obtained by performing calibration of $\Delta$Ct-n using the difference value, and the concentration of the target biomolecules may be obtained from the concentration of the external standard material according to the following equation using $\Delta$Ct-cal, thereby securing a result that reflects the difference between the binding affinity.

Concentration of external standard material x $2^{-\Delta Ct-cal}$

Therefore, it can be said that the target biomolecules in the sample to be analyzed are present at a concentration which is $2^{-\Delta Ct-cal}$ times higher than the concentration of the external standard material.

Further, the length of the design nucleic acid may be decided depending on the target biomolecules to thus represent the target biomolecules by the length of the design nucleic acid, thereby analyzing the corresponding target biomolecules. Preferably, qualitative and quantitative analyses of one or more target biomolecules may be simultaneously performed using the length of the amplified product of the design nucleic acid.

In order to analyze the standard material and one or more target biomolecules in a sample to be analyzed, containing the standard material having a known concentration, design nucleic acids which correspond thereto and which have different lengths are manufactured. The design nucleic acids are bonded to ligands to be bonded to the standard material and the target biomolecules, thus preparing analytical ligands. Each analytical ligand is reacted with the sample to be analyzed to harvest standard material and target biomolecule complexes. The design nucleic acids of the complexes that are harvested may be subjected to PCR using a pair of universal PCR primers as a template, thereby synthesizing amplified products. From the result of analysis of the length of the amplified product, the presence or absence of one or more target biomolecules, corresponding to the length of the amplified product, and the amount of the target biomolecules that are present may be decided using the result of analysis of the design nucleic acid of the standard material.

Preferably, the primer pair used for the PCR of the design nucleic acid of the complex is a pair of universal primers, and any one of the primers is labeled with a fluorescent material which is a labeling material, thus labeling the amplified product.

In the present invention, examples of the usable labeling material may include a fluorescent pigment selected from among biotin, Cy2, GFP, YO-PRO-1, YOYO-1, Calcein, FITC, FlourX, ALEXA 488, Rhodamine 110, ABI 5-FAM, Oregon Green 500, Oregon green 488, RiboGreen, Rhodamine Green, Rhodamine 123, Magnesium Green, Calcium Green, TO-PRO-1, TOTO-1, ABI JOE, BODIPY 530/550, DiI, BODIPY TMR, BODIPY558/568, BODIPY564/570, Alexa 546, TRITC, Magnesium Orange, Phycoerythrin R & B, Rhodamine Phalloidin, Calcium Orange, Pyronin Y, Rhodamine B, ABI TAMRA, Rhodamine Red, Cy3.5, ABI ROX, Calcium Crimson, Alexa 594, Texas Red, Nile Red, YO-PRO-3, YOYO-3, R-phycocyanin, C-phycocyanin, TO-PRO-3, TOTO-3, DiD DilC(5), Thiadicarbocyainie, Cy5.5, Cy5, and Cy3.

Preferably, the size of the amplified product is decided using a gel electrophoresis or capillary electrophoresis method, whereby whether or not the target biomolecules are present and the amount of the target biomolecules that are present may be analyzed.

In order to analyze one or more target biomolecules in the sample to be analyzed using the design nucleic acid and the standard material, the present invention provides a ligand-nucleic-acid analysis method, kit, and apparatus in consideration of the type of standard material and the method of harvesting biomolecule-ligand complexes.

Preferably, in order to analyze the target biomolecules according to a nucleic acid analysis technique using the standard material for quality control and measurement in the sample including biomolecules, the present invention provides a ligand-nucleic-acid analysis method, kit, and apparatus. The ligand-nucleic-acid analysis method includes the steps of: manufacturing an analytical sample for analyzing the target biomolecules from the sample, manufacturing the ligand and the design nucleic acid of the standard material or the target biomolecules, harvesting complexes of the standard material or the target biomolecules formed by reacting the ligand and the design nucleic acid with the analytical sample in one tube, amplifying the design nucleic acids of the harvested complexes of the standard material or the target biomolecules to thus analyze the base sequence or the length of the amplified product, and analyzing the target biomolecules from the result of analysis of the amplified product.

In order to perform more efficient measurement in the method of analyzing the biomolecules according to the present invention, there is provided an apparatus for analyzing the biomolecules using a ligand PCR, which includes a sample treatment apparatus for preparing a sample to be analyzed, a module for manufacturing a standard material and also an analytical-ligand-biomolecule-harvest-ligand complex by reacting the sample with an analytical ligand, a harvest ligand, and the standard material and for amplifying the design nucleic acid contained in the complex and the standard material, and a module for analyzing the amplified product. The apparatus and the system of the present invention may include the sample treatment apparatus including mixing, dissolution, and reaction chambers and an amplification apparatus, which are integrally operated.

The apparatus and the system for analyzing the biomolecules using the ligand-nucleic-acid analysis method are a ligand-nucleic-acid analysis apparatus and a ligand-nucleic-acid analysis system which are used to perform the method of analyzing the design nucleic acid using nucleic acid amplification and analysis techniques. The apparatus and the system include a sample treatment apparatus for preparing the biomolecules in the sample including the target biomolecules, a module for manufacturing an analytical-ligand-biomolecule-harvest-ligand complex and a standard-analytical-ligand-standard-material-standard-harvest-ligand complex by reacting the sample with an analytical ligand, a harvest ligand, a standard analytical ligand, and a standard harvest ligand, for cleaning the complexes, and for amplifying the design nucleic acids of the complexes, and a module for analyzing the amplified product.

The analysis of a sample used to analyze the biomolecules of a target involves a series of sample preparation steps and is performed in the sample treatment apparatus including the mixing chamber and the dissolution chamber. These steps may include filtration, cytolysis, and mixing of nucleic acids with reagents.

According to a preferable embodiment of the present invention, the biomolecules may be preferably manufactured by solubilizing cells in a biosample including cells, and the additional steps may be performed using a known method.

Preferably, in a method of separating an analytical ligand-biomolecule complex and a standard analytical ligand-standard material manufactured using the plate used in the ELISA, in the case of the sample, after the target biomolecules and the standard material are bonded to supports, the analytical ligands may be reacted to manufacture the biomolecule-analytical-ligand complex, followed by cleaning. Thereby, the non-bonded analytical ligands are removed, thus preparing purified complexes.

The analytical sample, the analytical ligand, the harvest ligand, the standard analytical ligand, and the standard harvest ligand may be reacted to manufacture an analytical-ligand-biomolecule-harvest-ligand complex and a standard-analytical-ligand-standard-material-standard-harvest-ligand complex. The complexes manufactured using various methods may be harvested by a magnet due to the magnetism of the harvest ligand and the standard harvest ligand, followed by cleaning, whereby the non-bonded analytical ligand and standard analytical ligand are removed, thus preparing the purified complexes.

The design nucleic acids of the standard-analytical-ligand-standard-material-standard-harvest-ligand complex and the analytical-ligand-biomolecule-harvest-ligand complex that are separated may be quantitatively analyzed using a typical nucleic acid analysis method. In addition, from the analyzed results, the biological meaning of the target biomolecules in the biosample containing the target biomolecules may be decided.

Examples of the sample may include cultured cells, proteins, nucleic acids, or biological drugs containing them, tissue which is obtained from the group consisting of cells, spores, microorganisms, and viruses or from the testis, or body fluid such as blood, plasma, serum, urine, saliva, sweat, semen, and mucus, but are not limited thereto. The examples are considered to contain the selected target biomolecules, and the target sample contains two or more target biomolecules. The method includes introducing the sample into an apparatus including the mixing chamber for mixing the sample and a sample preparation control group. The sample preparation control group is selected from the group consisting of proteins, cells, spores, microorganisms, and viruses, and includes a material for quality control. The apparatus also has the dissolution chamber and the reaction chamber. The sample is mixed with the sample preparation control group in the mixing chamber.

The reaction mixture in the reaction chamber of the reaction vessel of the amplification apparatus is exposed to amplification conditions of the design nucleic acid. Amplification of RNA or DNA templates using a reaction is known [U.S. Pat. No. 4,683,195; U.S. Pat. No. 4,683,202; PCR Protocols: A Guide to Methods and Applications (Innis et al., 1990)]. Amplification of the nucleic acid of DNA involves repetition of cycles which include thermally denaturing the DNA, annealing two oligonucleotide primers to the sequence that is adjacent to the DNA segment to be amplified, and extending the annealed primers using a DNA polymerase. The primers are crossed to opposite strands of the target sequence, and are oriented so that DNA synthesis using the polymerase is performed across the region between the primers, efficiently doubling the amount of DNA segments. Further, since extension products may be complementary and may be bonded to the primers, the successive cycles serve to substantially double the amount of DNA synthesized during the previous cycle. This leads to the exponential accumulation of specific target segments at a ratio of 2n per cycle (n being the number of cycles). Methods such as an amplification reaction and a ligase chain reaction (LCR) may be used to directly amplify a nucleic acid sequence of a target DNA sequence. An isothermal amplification reaction is also known and may be used according to the method of the present invention.

The amplification reaction of the design nucleic acid is preferably performed using heat treatment equipment which heats and/or cools the reaction mixture in the reaction vessel to the temperature required for the amplification reaction. The heat treatment equipment may include one or more detection tools for detecting the nucleic acid sequence of the standard material of the sample preparation control group and the nucleic acid sequences of one or more standard substances for testing in the sample. Preferable heat treatment equipment including an optical detector for amplifying and detecting the nucleic acid sequence in the reaction vessel may be used (U.S. Pat. No. 6,369,893; U.S. Pat. No. 6,391,541). Further, there are many other known methods suitable for use in the present invention in order to control the temperature of the reaction mixture and to detect the nucleic acid sequence in this reaction mixture.

In another preferable method for detection of amplified products, a fluorescent probe includes oligonucleotides labeled by fluorescent reporter dyes. The cleavage of a capture probe results in an increase in the fluorescent intensity of the reporter dye.

A fluid control apparatus may be controlled using a computer according to a desired protocol. When a single valve is used, since there is only one failure factor, high manufacturing yields may be obtained. The integration of fluid control and treatment constitution membranes allows a compact apparatus (e.g. in the form of a small cartridge) to be realized and facilitates automation of molding and assembly. The above-described system may advantageously have dilution and mixing capabilities, intermediate cleaning capabilities, and reliable pressurization capabilities. The fluid path in the system is normally closed so as to minimize contamination of the fluid in the system and to facilitate reception and control of the fluid. The reaction vessel is conveniently separable and interchangeable and, in some examples, disposable.

Chronic diseases including cancer are diagnosed using blood, which is a biosample. Particularly, the diagnosis of cancer has a limitation in that 5 to 6 biomolecules for monitoring patients are tested and only the cut-off value of the result thereof is used. Chronic diseases are caused by various gene mutations, and cancer is a representative disease among the chronic diseases. There is a limit to the extent to which cancer can be efficiently screened using a single index. Accordingly, in practice, only about 30 to 40% of cancer patients are found using conventional blood tests.

An *in-vitro* diagnostic multivariate index assay (IVDMIA) is a diagnosis method for quantitatively analyzing the functional relation between a normal group and a cancer group based on the results of quantitative examination of various target biomolecules. In the assay, there is used an apparatus for combining a plurality of results in order to classify, score, and index the patient's specific results using analytical functions for the purpose of diagnosing, curing, alleviating, treating, and preventing diseases or other conditions which are biological meaning.

In the present invention, a clinical decision support system for determining the biological meaning based on the biomolecule analysis information generated using the ligand-nucleic-acid analysis method is exemplarily selected as a high risk group for diagnosis and disease, without limitation thereto.

In order to accomplish the above object, the present invention provides a method of determining the biological meaning of target biomolecules in a sample or a patient, from which the sample is collected, using the result of analysis of a standard material and target biomolecules according to ligand-nucleic-acid analysis, and a clinical decision support system for determining the biological meaning of the target biomolecules according to the ligand-nucleic-acid analysis. The method includes (A) generating the result of analysis of the target biomolecules using a standard material for measurement while performing quality control using the standard material in the sample according to the ligand-nucleic-acid analysis, (B) transforming the result of analysis of the biomolecules using a partial dependency plot or a partial dependency function relation of an ensemble technique using a decision tree to thus generate the transformed result of analysis of the biomolecules, and (C) inputting the transformed result of analysis of the biomolecules into a predetermined biological meaning determination model to thus generate biological meaning information for each sample.

Preferably, the biological meaning determination model is a logistic regression model.

Preferably, in the logistic regression model, a ridge penalty function is used.

Preferably, the ensemble technique is any one method of boosting and random forest.

The biological meaning determination information further produces information on the contribution of the target biomolecule to biological meaning determination. In the case of the contribution of the target biomolecule to biological meaning determination, it is preferable to provide the extent of influence on the biological meaning of one or more target biomolecules included in the biological meaning determination model using a predetermined discrimination function obtained according to a logistic model.

Preferably, the contribution of the target biomolecule to disease biological meaning determination is provided in the form of a coefficient plot.

In a biological meaning analysis kit including a target biomolecule antibody, preferably, the analysis is performed in a third system for generating analysis information on the target biomolecule, or is performed in a biological meaning determination system which is connected to the third system via a wired-wireless network and which receives the analysis information on the target biomolecule from the third system.

In order to accomplish the above object, the present invention provides a method of generating a biological meaning determination model using target biomolecules based on transformed analysis information on the target biomolecule. The method includes (A) generating analysis information on each of multiple types of target biomolecules from blood, plasma, or serum of subjects including a plurality of patients and a plurality of normal persons, or materials sampled from a subject's body, followed by predetermined transformation of the analysis information that is generated, (B) generating a plurality of classifiers using a decision tree based on the transformed analysis information on the target biomolecule with a selected one of the subjects as a model generation subject group, and generating a plurality of biological meaning determination model candidates using one or more target biomolecules by combining the plurality of classifiers that are generated, (C) inputting the transformed analysis information on the target biomolecule of a model verification subject of a model verification subject group including the subject who is not included in the model generation subject group into the biological meaning determination model candidate and generating biological meaning determination information on each model verification subject, and (D) performing a predetermined evaluation of the biological meaning determination information and selecting a biological meaning determination model that satisfies a predetermined evaluation index.

Preferably, the analysis information may further include analysis-result ratio information on at least one pair of target biomolecules.

In order to perform the predetermined transformation, it is preferable to transform the analysis information using a partial dependency plot or a partial dependency function relation of an ensemble method using a decision tree.

Preferably, the biological meaning determination model is a logistic regression model.

Preferably, the predetermined evaluation index is at least one of precision, specificity, sensitivity, and an ROC curve area.

Preferably, the ensemble technique is any one method of boosting and random forest.

The biological meaning determination information further produces information on the contribution of the target biomolecule to biological meaning determination. In the case of the contribution of the target biomolecule to biological meaning determination, it is preferable to provide the extent of influence of one or more target biomolecules included in the biological meaning determination model on cancer using a predetermined discrimination function obtained according to a logistic model.

In order to accomplish the above object, the present invention provides a biological meaning determination prediction system for predicting biological meaning determination directly using an analytical kit or using analysis information obtained using the analytical kit, and a clinical decision support system for determining the biological meaning. The biological meaning determination prediction system includes (A) an information-obtaining module for obtaining analysis information or analysis-result ratio information on the target biomolecule constituting the target biomolecule combination measured from blood, plasma, or serum of a subject, or samples collected from a subject's body, (B) one or more transformation modules which are set in advance based on the analysis information or the analysis-result ratio information, and a biological meaning determination prediction module for treating the obtained analysis information or analysis-result ratio information using a predetermined biological meaning determination prediction model, and (C) a biological meaning determination prediction information generation module for generating at least one biological meaning determination prediction information from the biological meaning determination prediction module. The transformation module first generates analysis-result transformation information on the analysis information or first generates the analysis-result ratio transformation information on the analysis-result ratio information. The biological meaning determination prediction model receives the analysis-result transformation information or the analysis-result ratio transformation information as an input value.

Preferably, examples of a method of obtaining the analysis information or the analysis-result ratio information on the target biomolecule using the information-obtaining module include one or more methods among (A) a method of directly obtaining the information from the biological meaning analytical kit using the biological meaning prediction system, (B) a method of obtaining the information by receiving the information that is transmitted from the third system for producing the analysis information on the target biomolecule in an analytical kit connected through a wired-wireless network to the biological meaning determination prediction system, and (C) a method of obtaining the information by receiving the information that is transmitted from a computer of a person obtaining the analysis information on the target biomolecule, the computer being connected through a wired-wireless network to the biological meaning determination prediction system.

Preferably, the transformation module generates the analysis-result transformation information or the analysis-result ratio transformation information using a partial dependence plot or a partial dependency function relation of an ensemble technique using a tree.

Preferably, the biological meaning determination prediction model is a logistic model, and the logistic model is a predetermined functional formula for estimating a probability value classified as cancer by receiving the analysis-result transformation information or the analysis-result ratio transformation information.

The biological meaning determination prediction information generation module further generates information on the contribution of the target biomolecule to disease analysis. In the case of the contribution of the target biomolecule to disease analysis, it is preferable to provide the extent of influence of the target biomolecules, which are included in a target biomolecule combination, on cancer in the form of a coefficient plot using a predetermined discrimination function obtained according to a logistic model.

In order to accomplish the above object, the present invention provides a method of processing a variable value for the target biomolecule, associated with a biological information decision statistical model for processing the target biomolecule information including two or more target biomolecules for biological meaning determination. The method includes (A) obtaining an original variable value for the target biomolecule in each of two or more samples, (B) performing predetermined processing using the original input variable value for the target biomolecule to thus form a partial dependence plot or a partial dependence function relation for the target biomolecule, (C) generating a transformation variable value for the target biomolecule with respect to the original variable value for the target biomolecule using the partial dependence plot or the partial dependence function relation for the target biomolecule, and (D) using the transformation variable value for the target biomolecule to generate a predetermined biological meaning determination statistical model or to perform the biological meaning determination statistical model. The partial dependence plot or the partial dependence function relation is obtained using an ensemble technique, and the ensemble technique is at least one technique of a boosting algorithm technique and a random forest algorithm technique.

Preferably, the partial dependence plot or the partial dependence function relation for the target biomolecule are formed so that the original variable values are averaged with respect to target biomolecules other than the above-described target biomolecule among the target biomolecules constituting the above-described target biomolecules.

Preferably, the original variable value is at least one of the analysis information on the target biomolecule and the extent of analysis-result ratio of two or more target biomolecules.

In order to accomplish the above object, the present invention provides a method of processing influence information on the target biomolecule, associated with a biological meaning determination statistical model for processing the target biomolecule information including two or more target biomolecules for biological meaning determination. The method includes (A) generating influence information on individual target biomolecules constituting the target biomolecules, and (B) generating information for visualizing the influence information on the individual target biomolecules constituting the target biomolecules. The influence of the individual target biomolecules is determined using a discrimination function obtained from a logistic model. The discrimination function is represented by the following [General Formula 2], the logistic model has a value between 0 and 1, and a ridge function is used to estimate the regression coefficient included in the logistic model.

[General Formula 2]

$$\beta_0 + \beta_1 g_1(X_1) + \beta_2 g_2(X_2) + \cdots + \beta_k g_k(X_k)$$
$$= \beta_0 + \beta_1(g_1(X_1) - \overline{g_1}) + \beta_2(g_2(X_2) - \overline{g_2}) + \cdots + \beta_k(g_k(X_k) - \overline{g_k})$$

In g (x), it is preferable to use the transformation variable value for the target biomolecule with respect to the original variable value for the target biomolecule using the partial dependence plot or the partial dependency function relation.

Preferably, the visualization is shown using a two-dimensional plane chart or graph.

Further, the present invention provides a system for analyzing a biological meaning using biomolecule analysis information produced according to a ligand-nucleic-acid analysis method, and a method and a clinical decision support system for determining the biological meaning. The system includes (A) a module for receiving the biomolecule analysis result using clinical information of a control group as a standard and then building a database, (B) a module for performing pre-processing in the analysis system using the input biomolecule analysis result and a module for generating a patient model using the result of pre-processing, (C) a module for loading the generated model and applying the model to a patient visited to thus perform diagnosis which is a blind test, and (D) a module for evaluating the performance of the system via cross validation.

Many biomolecule analysis results generated according to the present invention include a great amount of information having high-dimensional characteristics, and may produce much information related to cells, tissues, or diseases. The module that performs the pre-processing in the analysis system using the input data serves to find the features that affect the patient's condition, and a multivariate analysis method of improving the classification performance is a feature selection procedure for finding important variables for accurate treatment and diagnosis, thus reducing the dimension or transforming the characteristics, thereby finding important features.

Specific examples of feature extraction include an unsupervised learning method and a supervised learning method, depending on whether or not class information is used for learning. In PCA (principal component analysis) or ICA (independent component analysis), which is mainly used in the unsupervised learning method, features may be extracted in consideration of the characteristics of variables. The supervised method is a method of selecting variables using the statistical significance between class information and variables or the correlation between variables. In this method, the main features may be calculated using the performance applied to the classifier by sequentially adding or removing the features in forward or backward directions in a given feature set.

The module for performing the learning using the result of pre-processing to thus generate the patient model is a procedure for classifying the selected features into individual classes using an appropriate classifier.

In the present invention, an artificial neural network is used. Having characteristics of deciding behaviors depending on inputs and outputs, the artificial neural network is applied to various fields such as pattern recognition, function approximation, and classification technique. The artificial neural network has a structure that includes several layers, nodes, and connection weights between neural networks. The neural network interlayer connection scheme used in the present invention is a feed-forward scheme. Based on the input pattern, the neural network connection weights for each node and the activation function are used to calculate the output value. When the estimated value is different from the actual result value during processing of some tasks using the generated combination information, a process of comparing the calculated value with the actual result value in order to reduce the error is repeated, thereby finding the values.

In the method of generating the patient model, there are provided a method and an apparatus for analyzing biomolecules, selected from the group consisting of linear models, support vector machines, neural networks, classification and regression trees, ensemble learning methods, discriminant analysis, nearest-neighbor methods, Bayesian networks, and independent component analysis.

Case-based diagnosis is of great importance for the majority of diseases for which an accurate and comprehensive mechanism has not been established. However, the conventional case-based machine-learning inference system designed on the basis of only specific machine-learning techniques has low precision and accordingly the development of an improved system is continuously required. Further, the conventional system is designed only as a disease discriminator using all learned clinical test items, and does not provide a method of utilizing importance or priority of clinical test items for each disease.

The present invention relates to a system for diagnosing diseases and selecting test items using case-based machine learning inference for supporting accurate disease diagnosis by a physician. Further, the present invention relates to a system for discriminating a disease by analyzing test information of a new patient using a disease discriminator, which is a machine-learning device, on the basis of an artificial neural network trained using a case database of patients and for selecting the least important test item for final discrimination of each disease using preliminary diagnosis to thus provide the test items. A disease diagnosis technique using machine-learning inference includes individual application of machine-learning techniques. Examples of the method of generating the patient model include linear models, support vector machines, neural networks, classification and regression trees, ensemble learning methods, discriminant analysis, nearest-neighbor methods, Bayesian networks, and independent component analysis.

The constitution and practical application of the present system may be divided into two types. One is a standalone diagnostic system for diagnosis only, and the other is an integrated system that is linked with a conventional hospital information system, that is, OCS, PACS, and LIS. For linking with the integrated system, the system must be constituted according to HL7 and DICOM protocol. The present system is an initial model and is linked with a PMS (patient monitoring system) to increase the precision of diagnosis. Further, the present system may be developed as a system linked with various medical information systems such as OCS, EMR, and PACS in addition to PMS, thereby being developed as a more accurate diagnosis system including various disease factors.

The present invention provides a method and apparatus for analyzing biomolecules using oligonucleotides. In the method and apparatus, the biosamples are one or more selected from the group consisting of bacteria, fungi, viruses, cell lines, and tissues.

**Advantageous Effects**

In the present invention, it is possible to confirm the biological meaning of biomolecules in a sample by analyzing two or more biomolecules using a ligand PCR, and it is also possible to amplify a signal indicating the presence of biomolecules using a PCR method, thus detecting the signal with high fluorescent sensitivity. Furthermore, the present invention provides a method of analyzing various biomolecules in a single test and efficiently determining the biomolecule changes in a sample, susceptibility to disease, diagnosis of disease, and differences between individuals, such as differences in susceptibility to diseases and in reaction to therapeutic agents, from the analyzed result using software.

**Description of Drawings**

FIG. 1 is a conceptual diagram of a ligand-nucleic-acid analysis method;

FIG. 2 is a conceptual diagram showing amplification and analysis of a design nucleic acid of the ligand-nucleic-acid analysis method;

FIG. 3 shows the result of ligand-nucleic-acid analysis of a standard material subjected to serial dilution using real-time PCR, and also the result of analysis of an amplified product of the design nucleic acid of a complex using a Taqman probe;

FIG. 4 shows the result of analysis of the length of the amplified product of the design nucleic acid representing the standard material and target biomolecules using a capillary electrophoresis method;

FIG. 5 shows the logical architecture of a biological meaning determination system;

FIG. 6 shows the structure of the biological meaning determination system;

FIG. 7 shows the structure of a biological meaning determination software;

FIG. 8 shows the role of an integrated management tool ACS in the biological meaning determination software;

FIG. 9 shows the data analysis module of biological meaning determination;

FIG. 10 shows the result of analysis of the target biomolecules produced using the ligand-nucleic-acid analysis method, and is a flowchart showing the determination of the biological meaning of a patient suffering from acute myocardial infarction or sepsis;

FIG. 11 is a flowchart showing the diagnosis of diseases for a blood sample of a specific person using a clinical decision support system for producing a biomolecule analysis result adopting a program that includes a database and an artificial neural network constituted for each disease group based on the produced analysis results; and

FIG. 12 shows an example of diagnosing a patient by analyzing the target biomolecule result, which is produced according to the ligand-nucleic-acid analysis, using the clinical decision support system for determining the biological meaning.

**Best Mode**

**Mode for Invention**

Hereinafter, a method of manufacturing a standard material for quality control and measurement and a design nucleic acid according to the present invention and a ligand PCR analysis method of biomolecules in a sample including one or more biomolecules using the same will be described in detail with reference to the accompanying drawings and Examples. The following embodiments are illustrative of the present invention and are not intended to limit the scope of the present invention.

Example 1. Target biomolecule and external standard material for quality control and measurement

It is required to find a diagnostic bio-index having high specificity and high sensitivity for accurate and rapid diagnosis of acute myocardial infarction in a patient, that is, to find an acute myocardial infarction specific marker.

Examples of well-known AMI diagnostic markers used to diagnose acute myocardial infarction (AMI) from the blood of a patient include myoglobin, CK-MB, Troponin I, and Troponin T. The rapid diagnosis of myocardial infarction is very important because myocardial infarction, particularly acute myocardial infarction, leads to death if not treated within 6 hours after chest pain occurs.

Sepsis may progress to severe sepsis accompanied by various organ dysfunctions, and severe cases thereof may lead to sepsis shock, which is indicative of refractory hypotension, which may be fatal. Therefore, rapid, sensitive, and specific markers are required for the diagnosis, severity assessment, and progression observation of sepsis. Inflammatory reactions caused by infection or tissue damage are a series of processes over time, beginning locally and releasing interleukin (IL)-6 and cytokines and generating acute phase reactants in the liver. The role of objective markers as well as clinical symptoms or signs is important in the diagnosis of inflammation and infection. Examples of markers that are known to date may include leukocyte count (including neutrophils or immature fractions), C-reactive proteins (CRP), procalcitonin (PCT), IL-6, IL-10, or TNF-$\alpha$.

The target biomolecules used in the present invention are myoglobin (Life Technologies, USA), CK-MB (Life Technologies, USA), Troponin I (Life Technologies, USA), or Troponin T (Life Technologies, USA), which are indices of acute myocardial infarction, and proteins such as C-reactive protein (CRP; Life Technologies, USA), procalcitonin (PCT; Life Technologies, USA), IL-6 (Life Technologies, USA), and IL-10 (Life Technologies, USA), which are indices of sepsis.

Further, the standard material for quality control and measurement is *E. coli* Enoyl-ACP Reductase (Sino Biological Inc., China), which is an Escherichia coli protein, and Phototropin 2 (Cosmo Bio Co. Ltd. Japan), which is a plant protein.

Example 2. Preparation of design nucleic acid and Taqman probe

In order to analyze one or more target biomolecules in a sample having a standard material for quality control and measurement according to a ligand-nucleic-acid analysis method using a ligand and a design nucleic acid corresponding thereto, in a method proposed by the present invention, the basic constitution of the design nucleic acid is represented by the following General Formula (I):

$$5\text{'-}N\text{-}P1\alpha\text{-}N_V\text{-}P3\beta\text{-}3\text{'} \qquad (I)$$

In General Formula (I), a P1 region is a portion completely complementarily bonded to a forward primer, and has a portion which includes a reactive group N having an amine group at a 5' end thereof and which is connected to the ligand, which is a material binding to the target biomolecule. Preferably, the design nucleic acid may have a spacer including about 10 nucleotides at the upstream of a base sequence portion that is complementary to the forward primer. This spacer has a stereoscopic effect of the ligand bonded to the structure of General Formula (1), which easily allows the forward primer to bind to the P1 region, and the base sequence of the spacer is 5'-TGATTGAATT-3'.

An $N_V$ region is a base sequence representing a specific biomolecule (or an external standard material) bound to the ligand by a specific base sequence or a length thereof, and serves to detect and analyze a specific biomolecule bound to the ligand. Preferably, the length thereof is 10 to 200 nucleotides.

A P3 region is a portion complementary to the backward primer.

$\alpha$ and $\beta$ denote the number of nucleotides, and $\alpha$ and $\beta$ are an integer of 8 to 30.

Preferably, the forward primer and the backward primer may be a pair of universal primers having a common base sequence in all of the design nucleic acids.

The primer pairs to be used in the present Example are shown in [Table 1].

# EP 3 287 530 A1

As indicated in [General Formula (I)], the design nucleic acid, which is designed in consideration of the reactive group, the spacer and the universal primer pair, is shown in [base sequence (I)].

5'-NH$_2$-TGATTGAATT-CGGAAGCGTGCTGGGCC N$_V$ CATAACCCAGAGGTCGA-3' [(base sequence (I)]

CGGAAGCGTGCTGGGCC is specified as sequence No. 1, and CATAACCCAGAGGTCGA is specified as sequence No. 2.

[Table 1] Base sequence of universal primer pair

| Primer | Base sequence (5' → 3') | Length (nt) | Sequence No. |
|---|---|---|---|
| Forward primer | 5'-CGGAAGCGTGCTGGGCC-3' | 17 | 3 |
| Backward primer | 5'-TCGACCTCTGGGTTATG-3' | 17 | 4 |

The base sequence of the N$_V$ region of the design nucleic acid, which represents the target biomolecule and the external standard material (*E. coli* Enoyl-ACP Reductase) as the specific base sequence, is shown in [Table 2].

[Table 2] Base sequence of N$_V$ region of design nucleic acid representing target biomolecule and external standard material

| Protein | Base sequence (5' → 3') | Length (nt) | Sequence No. |
|---|---|---|---|
| Standard material | GATTTCTATTGATGCTCGCTCCAACCGACAGATTGAGATT | 40 | 4 |
| Myoglobin | GATTTGTATTACGTGAAAACGGCAACAATTGATTGAGAT | 40 | 3 |
| Creatine Kinase Isoform MB | GATTTGTACCACTACCAGGAGGACCCTGTATTTTGATTG A | 40 | 6 |
| Cardiac Troponin I | GATTTGTATTGACGGGCGAACTGCACCAGTTGAGATTAAA | 40 | 7 |
| Cardiac Troponin T | GATTTGTATTTCAGGTACTCCGAGGCGTCCTGATTGAGAT | 40 | 8 |
| IL-10 | GATTTGTATTGATGCTCGCTCCAACCGACAGATTGAGATT | 40 | 9 |
| IL-6 | GATTTGTATTACGTGAAAACGGCAACAATTGATTGAGAT | 40 | 10 |
| CRP (C-reactive protein) | GATTTGTACCACTACCAGGAGGACCCTGTATTTTGATTG A | 40 | 11 |
| PCT (Procalcitonin) | GATTTGTATTGACGGGCGAACTGCACCAGTTGAGATTAAA | 40 | 12 |

The structure and the base sequence of the Taqman probe which serves to recognize the N$_V$ region of the design nucleic acid and to generate a signal in order to analyze the external standard material and the target biomolecules are shown in [Table 3].

[Table 3] Taqman probe for analyzing base sequence of design nucleic acid representing external standard material and target biomolecule

| Taqman probe | Base sequence (5' → 3') | Length (nt) | Sequence No. |
|---|---|---|---|
| Standard material | Cy5.5-AGTCGGTTGGAGCGAGCATC-BHQ2 | 20 | 13 |
| Myoglobin | HEX-ARTTGTTGCCGTTTTCACGT-BHQ1 | 21 | 14 |
| Creatine Kinase Isoform MB | FAM-AATACAGGGTCCTCCTGGTAGTGG-BHQ1 | 24 | 15 |
| Cardiac Troponin I | ROX-CTGGTGCAGTTCGCCCG-BHQ2 | 17 | 16 |

18

(continued)

| Taqman probe | Base sequence (5' → 3') | Length (nt) | Sequence No. |
|---|---|---|---|
| Cardiac Troponin T | Cy5-AGGACGCCTCGGAGTACCTGA-BHQ2 | 21 | 17 |
| IL-10 | HEX-AATTGTTGCCGTTTTCAGCGT-BHQ1 | 20 | 18 |
| IL-6 | FAM-AATACAGGGTCCTCCTGGTAGTGG-BHQ1 | 21 | 19 |
| CRP | ROX-CTGGTGCAGTTCGCCCG-BHQ2 | 24 | 20 |
| PCT | Cy5-AGGACGCCTCGGAGTACCTGA-BHQ2 | 17 | 21 |

The base sequence and the length of the $N_V$ region of the design nucleic acid, which represents the target biomolecule and the external standard material (*E. coli* Enoyl-ACP Reductase) by the length of the nucleic acid, are shown in [Table 4].

[Table 4] Base sequence and length of $N_V$ region of design nucleic acid, which represents target biomolecule and standard material for quality control and measurement by length of design nucleic acid

| Protein | Base sequence (5' → 3') | Length (nt) | Sequence No. |
|---|---|---|---|
| Standard material | GATTTGTATTGATTGAGATTAAAGTG | 26 | 22 |
| Myoglobin | GATTTGTATTGATTGAGATTAAAGTGAAATG | 31 | 23 |
| Creatine Kinase Isoform MB | GATTTGTATTGATTGAGATTAAAGTGAAATGAATGA | 36 | 24 |
| Cardiac Troponin I | GATTTGTATTGATTGAGATTAAAGTGAAATGAATGAATGAT | 41 | 25 |
| Cardiac Troponin T | GATTTGTATTGATTGAGATTAAAGTGAAATGAATGAATGATGAAAT | 46 | 26 |
| IL-10 | GATTTGTATTGATTGAGATTAAAGTGAAATGAATGAATGATGAAATTGAAA | 51 | 27 |
| IL-6 | GATTTGTATTGATTGAGATTAAAGTGAAATGAATGAATGATGAAATTGAAAGGATT | 56 | 29 |
| CRP | GATTTGTATTGATTGAGATTAAAGTGAAATGAATGAATGAATGATGAAATTGAAAGGATTAAAGT | 61 | 29 |
| PCT | GATTTGTATTGATTGAGATTAAAGTGAAATGAATGAATGATGAAATTGAAAGGATTA AAGTGAAGT | 66 | 3C |

EP 3 287 530 A1

Example 3. Preparation of analytical ligand, harvest ligand, standard analytical ligand, and standard harvest ligand

Two types of monoclonal antibodies against different epitopes of the target biomolecule or polyclonal antibodies binding to the target biomolecule are divided into two groups. One group is coupled to the design nucleic acid and the other group is coupled to a magnetic material. The former and the latter are referred to as an analytical ligand and a harvest ligand, respectively. Further, in the case of the antibody of the standard material, an antibody coupled to the design nucleic acid is referred to as a standard analytical ligand, and an antibody coupled to a magnetic material is referred to as a standard harvest ligand.

Examples of the antibody against the target biomolecule used in the present invention include an anti-myoglobin polyclonal antibody (Life Technologies, USA), an anti-CK-MB polyclonal antibody (Life Technologies, USA), an anti-troponin I polyclonal antibody (Life Technologies, USA), and an anti-troponin T polyclonal antibody (Life Technologies, USA), which are indices of acute myocardial infarction, and an anti-reactive C-protein polyclonal antibody (Life Technologies, USA), an anti-procalcitonin polyclonal antibody (Life Technologies, USA), an anti-IL-6 polyclonal antibody (Life Technologies, USA), and an anti-IL-10 polyclonal antibody (Life Technologies, USA), which are indices of sepsis.

Examples of the antibody against the external standard material for quality control and measurement used in the present invention include a polyclonal *E. coli* Enoyl-ACP Reductase antibody (Sino Biological Inc., China) and a polyclonal Phototropin 1 antibody (Cosmo Bio Co. Ltd., Japan).

In the case of the design nucleic acid, the base sequences shown in [General Formula (1)] and [base sequence (I)] of Example 2 were ordered to manufacture oligomers (Bioneer, Korea).

In order to analyze the amplified product of the design nucleic acid using the Taqman probe, nine types of oligomers having an amine group were ordered and manufactured as the design nucleic acid shown in Example 2. One type represents an external standard material for quality control and measurement and eight types of oligomers represent eight types of target biomolecules, and they are shown in [Table 2].

In order to analyze the amplified product of the design nucleic acid using the length of the design nucleic acid, nine types of oligomers having an amine group were ordered and manufactured as the design nucleic acid shown in Example 2. One type represents an external standard material for quality control and measurement and eight types of oligomers represent eight types of target biomolecules, and they are shown in [Table 4].

Each design nucleic acid was bonded to a specific ligand (antibody) of the target biomolecule or a specific ligand (antibody) of the external standard material for quality control and measurement using a Thunder-Link oligo conjugation system (Innova Biosciences Ltd., UK) according to the protocol thereof.

100 $\mu$l of manufactured 60~100 M oligomer was added to an oligo activation reagent tube, and 100 $\mu$l of 1 mg/ml antibody was added to an antibody activation reagent tube and reacted at room temperature for 1 hour to activate it. The activated reaction solution was desalted using a prepared column, and the desalted design nucleic acid and antibody were mixed at an appropriate ratio and reacted at room temperature for 12 to 24 hours. A conjugate clean-up reagent was added to the design nucleic acid-antibody reaction solution, reacted for 10 minutes, and centrifuged at 15,000 g for 5 minutes, thus harvesting a design nucleic acid-antibody structure. The conjugate clean-up reagent was further added to the harvested design nucleic acid-antibody structure and was centrifuged, thus harvesting a purified design nucleic acid-antibody structure.

The 18 types of harvested design nucleic acid-antibody structures manufactured as described above are referred to as an analytical ligand when the antibody is the antibody of the target biomolecule and are referred to as a standard analytical ligand when the antibody is the antibody of the standard material for quality control and measurement.

In order to analyze the amplified product of the design nucleic acid using the Taqman probe, the magnetic material was bound to antibodies of eight types of target biomolecules, and an antibody of one type of the standard material for quality control and measurement using a Dynabeads antibody coupling kit (Life Technologies, USA) according to the protocol thereof.

The antibody and Dynabeads M-270 Epoxy, which was a magnetic bead, were reacted at 37°C for 12 to 24 hours and then harvested using a magnet. Cleaning was performed using a process of adding a cleaning solution contained in a kit and performing re-suspension, followed by magnetic harvesting, thus removing the antibody that was not bound, thereby harvesting a purified Dynabead-antibody structure. The Dynabead-antibody structure thus harvested is a harvest ligand when the antibody is the antibody of the target biomolecule, and is a standard harvest ligand when the antibody is the antibody of the external standard material for quality control and measurement.

Example 4. Preparation of biosample for analysis

*E. coli* Enoyl-ACP Reductase, which was a standard material for quality control and measurement, and target biomolecules were prepared in TBS [Tris-buffered saline; 150 mM NaCl, 20 mM tris-Cl (pH 7.5), and 0.02% NaN$_3$] at a final concentration of 10 ng/ml in a PCR tube.

Analytical samples were prepared in various amounts (from 1 ng/ml to 1 ag/ml) in the PCR tube using serial dilution of the standard material and the target biomolecules.

Example 5. Formation of complexes and harvesting of complexes using magnets

45 $\mu$l of the analytical sample solution that was prepared above was placed in a well of a micro-titer plate (Falcon 3911; Becton Dickinson). 50 $\mu$l of TETBS (TBS containing 0.1 mM EDTA and 0.1% Tween 20) including 0.45% nonfat dried milk and denatured salmon sperm DNA (0.1 mg/ml) was added to the analytical sample solution.

10 $\mu$l of TETBS including eight types of analytical ligands, eight types of harvest ligands, one type of standard analytical ligand, and one type of standard harvest ligand, which were manufactured in Example 3 and which were mixed with each other in equal amounts, was added to the prepared analytical sample and allowed to react at room temperature for 30 minutes.

250 $\mu$l of TETBS was added, and a biomolecule complex and a standard material complex obtained using the magnet were cleaned several times, followed by final harvesting. The harvested complex design nucleic acid was subjected to nucleic-acid analysis in Example 7.

Example 6. Formation of complexes and harvesting of complexes using ELISA method

45 $\mu$l of the analytical sample that was prepared above was placed in a well of a micro-titer plate (Falcon 3911; Becton Dickinson). The plate with the analytical sample was treated at 4°C for about 15 hours to thus fix biomolecules to the well surface of the plate. The well of the plate was cleaned three times with 250 $\mu$l of TBS.

200 $\mu$l of ETBS (0.1 mM EDTA-containing TBS) containing 4.5% nonfat dried milk and denatured salmon sperm DNA (1 mg/ml) was added to each well and allowed to react at 37°C for 80 minutes. A process of adding 150 $\mu$l of TETBS to each well and allowing the same to react for 5 minutes was repeated seven times.

50 $\mu$l of TETBS containing eight types of analytical ligands, standard analytical ligands, 0.45% nonfat dried milk, and denatured salmon sperm DNA (0.1 mg/ml) was added to the well that was prepared, and was reacted at room temperature for 45 minutes. The process of adding 250 $\mu$l of TETBS to each well and reacting for 10 minutes was repeated fifteen times, thus removing ligands that were not bound. Each well was cleaned three times with $NaN_3$-free TBS, thus obtaining a purified complex. The obtained complex design nucleic acid was subjected to nucleic-acid analysis in Example 8.

Example 7. Analysis of biomolecules using base sequence of design nucleic acid

One or more target biomolecules were analyzed in a sample, to which a standard material for quality control and measurement was added, using the base sequence of the design nucleic acid according to a multiplex real-time polymerase chain reaction (multiplex real-time PCR) method using a Taqman probe corresponding to the base sequence of the design nucleic acid.

Real-time LightCycler™ (Roche, Switzerland) PCR and FastStart DNA master hybridization probe PCR mix (Roche, Switzerland) were used for the multiplex real-time polymerase chain reaction.

In the case of a PCR reaction solution, sterile distilled water was added to a primer-probe mix containing 2.4 $\mu$l of 25 mM $MgCl_2$, 2 $\mu$l of a 5 $\mu$mole/l forward primer, a 5 pmole/l backward primer, a 2.5 pmole/l probe, and 2 $\mu$l of a LightCycler DNA FastStart hybridization mix, and mixing was sufficiently performed so that the total volume was 18 $\mu$l. The resultant mixture was transferred to a glass capillary, and 2 $\mu$l of the harvested complex (standard curve) was dispensed. The total volume of reactants used to perform the polymerization chain reaction was 25 $\mu$l. Accordingly, the concentrations of the primer and the probe for detecting the target biomolecules and the standard material for quality control and measurement were 0.4 $\mu$M (12.5 pmole/25 $\mu$l) and 0.2 $\mu$M (62.5 pmole/25 $\mu$l).

[Table 5] Constitution and concentration of reactants used to perform real-time PCR

| Component | | Volume ($\mu$l) | Concentration |
|---|---|---|---|
| 10x LightCycler DNA FastStart hybridization mix | | 2.5 | 1X |
| Primer-probe mix | Primer (5 pmole/ul) | 2.0 | 0.4 $\mu$M |
| | Probe (2.5 pmole/ul) | | 0.2 $\mu$M |
| Nuclease-free sterile distilled water | | 11.0 | - |
| 25 mM MgCl2 | | 2.5 | |
| Harvested complex solution | | 2.0 | - |

(continued)

| Component | Volume ($\mu$l) | Concentration |
|-----------|-----------------|---------------|
| Total | 25.0 | - |

After steps of purifying and harvesting the complex were performed, the PCR was performed under the following conditions: (a) a pre-denaturing step, at 95°C for 5 minutes, and (b) a 40°C cycle, a cycle at 95°C for 15 seconds (denaturing step) and at 56°C for 15 seconds (annealing and extension step). The composition of the reactants used to perform the multiplex real-time polymerase chain reaction is shown in Table 5.

The multiplex real-time polymerase chain reaction method is a method in which the fluorescence of the real-time polymerase chain reaction is detected and quantified in real time every cycle using a DNA polymerase and a fluorescence resonance energy transfer (FRET) principle. The fluorescence was set to be displayed in a green channel (510 ± 5 nm) when FAM™ was developed on a real-time monitor, in a yellow channel (555 ± 5 nm) when Hex™ was developed, in a red channel (660 ± 10 nm) when Cy5™ was developed, in an orange channel (610 ± 5 nm) when ROX™ was developed, and in a crimson channel (712 log pass) when Cy5.5™ was developed. The fluorescence was observed in the green channel, the yellow channel, the red channel, the orange channel, and the crimson channel.

In each tube, the threshold value of the crimson channel was set to 0.04, the threshold values of remaining channels were set to 0.03, and a Ct (threshold cycle) value was confirmed. In the case when a peak was observed at the Ct value of less than 36, the case was read positive.

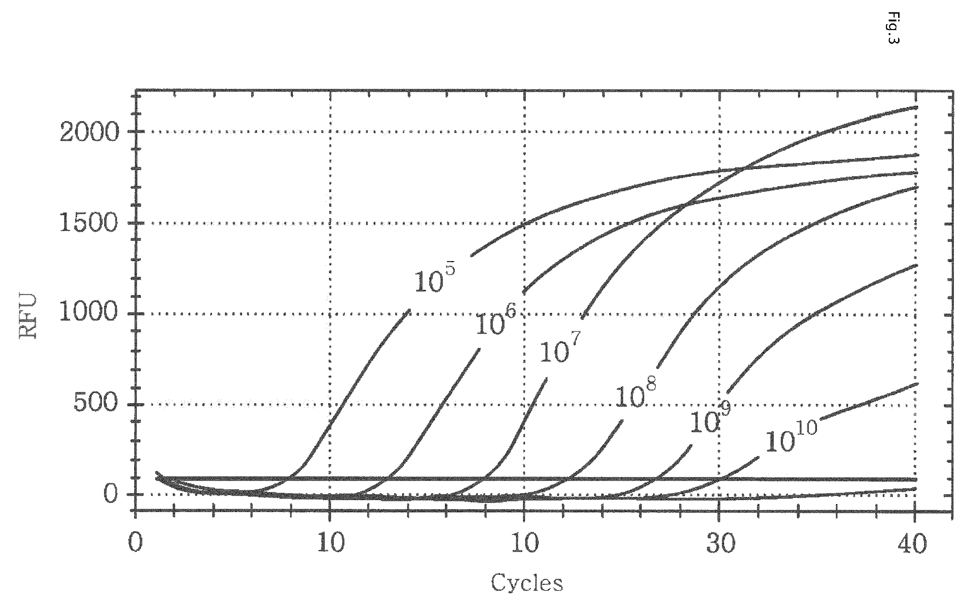

10 ng/ml *E. coli* Enoyl-ACP Reductase, which was a standard material for quality control and measurement, was diluted stepwise 10 times. The primer and the probe of the present invention were applied to the standard material sample that was manufactured above to perform the real-time PCR, thereby confirming a detection limit. The results are shown in FIG. 3.

For comparison with the result of the real-time PCR, the same standard material was diluted stepwise 10 times, and the primer and the probe of the present invention were then applied thereto, thereby confirming a detection limit using a conventional PCR. The conventional PCR showed a detection limit at a dilution magnification of up to $10^5$, but a multiplex real-time PCR showed a detection limit at a dilution magnification of up to $10^7$. Therefore, it could be seen that the detection limit was better in the multiplex real-time PCR than in the conventional PCR by $10^2$. FIG. 3 shows the result of the multiplex real-time PCR.

From the result of analysis of the curve that is obtained using real-time PCR of the design nucleic acid of the formed standard material complex in both cases where complexes formed during analysis of eight types of target biomolecules in the sample containing the standard material for quality control and measurement in one tube were harvested using a magnet and where the complexes were harvested using an ELISA method, it can be seen that the standard material and the target biomolecules are preferably analyzed in the sample to be analyzed using the ligand PCR according to the present invention.

Further, in both cases where the formed complexes were harvested using the magnet and using the ELISA method, the curve obtained from the real-time PCR of the design nucleic acid of the harvested target biomolecule complex and the curve corresponding to the standard material for quality control and measurement may be compared and analyzed, thus quantifying the target biomolecules or calculating the quantitative ratio of the standard material/target biomolecule.

In both cases where eight types of target biomolecule complexes for acute myocardial infarction and standard material complexes for quality control and measurement in one tube were harvested using a magnet and where the complexes were harvested using an ELISA method, the target biomolecules in the corresponding fluorescence channel were specifically confirmed using the real-time PCR for analysis of multiple biomolecules including a pair of primers and a probe for the complexes.

In order to evaluate the accuracy of the standard curve, the purified PCR product was serially diluted by 10 times ($10^{-4}$ to $10^{-9}$) to measure a within-run coefficient of variation (CV) and a between-run coefficient of variation.

The within-run coefficient of variation was obtained by dividing Myoglobin, which was the target biomolecule, and the ACT1 PCR product, which was the standard material, subjected to serial dilution at each concentration during one real-time PCR into three groups, followed by repeated measurement. The between-run coefficient of variation was analyzed using real-time PCR measurement of the standard material, Myoglobin, Creatine Kinase isoform MB, Cardiac Troponin I, Cardiac Troponin T, IL10, IL6, CRP, and PCT.

The standard curve was drawn by plotting a Cp value (x-axis) for a log value (y-axis) of an initial concentration of a template after serial dilution in a Light-Cycler (Roche Diagnostic Corp., Indianapolis, IN, USA). The slope and the error value were given, and the efficiency ($E = 10^{-1/\text{slope}}$) of the PCR was calculated using the slope.

The within-run coefficients of variation of the design nucleic acids of the complexes of Myoglobin for monitoring acute myocardial infarction and the standard material were obtained using the Ct value of the standard curve, which were 0.12 to 0.84% and 0.20 to 1.05%, and the mean standard deviation (SD) was 0.09 cycles (Table 6).

[Table 6] Within-run coefficient of variation of standard curve of standard material and target biomolecule by LightCycler real-time PCR

|  | Concentration (ng/ul) | Ct (Mean$\pm$SD) | CV (%) |
|---|---|---|---|
| Myoglobin (n=3) | $810^{-4}$ | $15.30\pm0.02$ | 0.12 |
|  | $810^{-5}$ | $18.60\pm0.08$ | 0.38 |
|  | $810^{-5}$ | $22.91\pm0.05$ | 0.20 |
|  | $810^{-7}$ | $25.22\pm0.22$ | 0.03 |
| E. coli Enoyl-ACP Reductase (n=3) | $1010^{-4}$ | $15.26\pm0.16$ | 0.05 |
|  | $1010^{-5}$ | $18.74\pm0.05$ | 0.29 |
|  | $1010^{-6}$ | $22.25\pm0.05$ | 0.20 |
|  | $1010^{-7}$ | $25.75\pm0.06$ | 0.23 |

The between-run coefficient of variation of the design nucleic acids of complexes of four types of target biomolecules, including Myoglobin, Creatine Kinase isoform MB, Cardiac Troponin I, and Cardiac Troponin T, for the acute myocardial infarction monitoring, and standard material was 0.07 to 0.92%, 0.12 to 0.55 %,

0.25 to 0.81%, 0.12 to 0.44%, 0.26 to 0.49%, and 0.05 to 0.29%. The mean standard deviation was 0.97 cycles. The PCR efficiency for design nucleic acids of the complex of the target biomolecule and design nucleic acid was 1.90 (Range, 1.60-2.14), 1.78 (Range, 1.65-1.88), 1.78 (Range, 1.65-1.88), 1.82 (Range, 1.67-2.00), and 1.80 (Range, 1.50-2.03), which did not show a significant difference between the design nucleic acids of the analyzed complexes (Table 7).

[Table 7] Between-run coefficient of variation and amplification efficiency of standard curve of target biomolecules by LightCycler real-time PCR

|  | Concentration (ng/ul) | Ct (Mean$\pm$SD) | CV (%) | Efficiency (Mean$\pm$SD) |
|---|---|---|---|---|
| E. coli Enoyl-ACP Reductase (n=20) | $810^{-4}$ | $15.26\pm0.16$ | 0.05 | 1.80$\pm$0.13 (Range, 1.50-2.03) |
|  | $810^{-5}$ | $18.74\pm0.05$ | 0.29 |  |
|  | $810^{-6}$ | $22.25\pm0.05$ | 0.20 |  |
|  | $810^{-7}$ | $25.75\pm0.06$ | 0.23 |  |
| Myoglobin (n=10) | $810^{-4}$ | $15.47\pm0.34$ | 0.17 | 1.90$\pm$0.23 (Range, 1.60-2.14) |
|  | $810^{-5}$ | $18.39\pm0.43$ | 0.12 |  |
|  | $810^{-6}$ | $22.89=0.47$ | 0.55 |  |
|  | $810^{-7}$ | $25.65\pm0.36$ | 0.43 |  |
| Creatine Kinase isoform MB (n=10) | $710^{-4}$ | $15.56\pm0.35$ | 0.31 | 1.78$\pm$0.07 (Range, 1.65-1.88) |
|  | $710^{-5}$ | $18.87\pm0.10$ | 0.81 |  |
|  | $710^{-6}$ | $22.66\pm0.15$ | 0.52 |  |
|  | $710^{-7}$ | $25.23\pm0.18$ | 0.25 |  |
| Cardiac Troponin I (n=10) | $810^{-4}$ | $15.42\pm0.25$ | 0.44 | 1.85$\pm$0.10 (Range, 1.69-2.03) |
|  | $810^{-5}$ | $18.13\pm0.05$ | 0.17 |  |
|  | $810^{-6}$ | $22.71\pm0.26$ | 0.36 |  |
|  | $810^{-7}$ | $25.04\pm0.38$ | 0.12 |  |

(continued)

| | Concentration (ng/ul) | Ct (Mean±SD) | CV (%) | Efficiency (Mean±SD) |
|---|---|---|---|---|
| Cardiac Troponin T (n=10) | $1010^{-4}$ | 15.93±0.09 | 0.34 | 1.82=0.07 (Range, 1.67-2.00) |
| | $1010^{-5}$ | 18.50±0.12 | 0.26 | |
| | $1010^{-6}$ | 22.13±0.21 | 0.26 | |
| | $1010^{-7}$ | 25.67±0.33 | 0.49 | |

The between-run coefficient of variation of the design nucleic acids of the complexes of four types of target biomolecules, including IL10, IL6, CRP, and PCT, for the sepsis monitoring, and the standard material was 0.25 to 0.48%, 0.32 to 0.52%, 0.29 to 0.46%, 0.22 to 0.45%, and 0.05 to 0.29%. The mean standard deviation was 0.97 cycles. The PCR efficiency for design nucleic acids of the complexes of the target biomolecule and standard material was 1.72 (Range, 1.54-2.07), 1.83 (Range, 1.62-2.27), 1.82 (Range, 1.59-2.05), 1.79 (Range, 1.52-2.27), and 1.80 (Range, 1.60-2.15), which did not show a significant difference between the design nucleic acids of the analyzed complexes (Table 8).

[Table 8] Between-run coefficient of variation of standard curve of target biomolecules by LightCycler real-time PCR

| | Concentration (ng/ul) | Ct (Mean±SD) | CV (%) | Efficiency (Mean±SD) |
|---|---|---|---|---|
| E. coli Enoyl-ACP Reductase (n=20) | $810^{-4}$ | 15.26±0.16 | 0.05 | 1.80±0.13 (Range, 1.60-2.15) |
| | $810^{-5}$ | 18.74±0.05 | 0.29 | |
| | $810^{-6}$ | 22.25±0.05 | 0.20 | |
| | $810^{-7}$ | 25.75±0.06 | 0.23 | |
| IL-10 (n=10) | $810^{-4}$ | 15.54±0.34 | 0.25 | 1.72±0.19 (Range, 1.54-2.07) |
| | $810^{-5}$ | 18.46±0.63 | 0.25 | |
| | $810^{-6}$ | 22.96±0.87 | 0.48 | |
| | $810^{-7}$ | 25.72±0.36 | 0.32 | |
| IL-6 (n=10) | $710^{-4}$ | 15.26±0.85 | 0.52 | 1.83±0.03 (Range, 1.62-2.27) |
| | $710^{-5}$ | 18.55±0.10 | 0.52 | |
| | $710^{-6}$ | 22.37±0.15 | 0.32 | |
| | $710^{-7}$ | 25.95±0.18 | 0.47 | |
| CRP (n=10) | $810^{-4}$ | 15.97±0.27 | 0.37 | 1.82±0.30 (Range, 1.59-2.05) |
| | $810^{-5}$ | 18.65±0.25 | 0.29 | |
| | $810^{-6}$ | 22.16±0.46 | 0.46 | |
| | $810^{-7}$ | 25.55±0.58 | 0.32 | |
| PCT (n=10) | $1010^{-4}$ | 15.65±0.19 | 0.37 | 1.79±0.16 (Range, 1.52-2.27) |
| | $1010^{-5}$ | 18.12±0.22 | 0.22 | |
| | $1010^{-6}$ | 22.05±0.15 | 0.37 | |
| | $1010^{-7}$ | 25.39±0.27 | 0.45 | |

In the present invention, the design nucleic acid in the complex harvested by performing simultaneous reaction of one or more target biomolecules with the standard material for quality control and measurement in one tube was analyzed using Multiplex Real-time PCR, thus obtaining a Ct value. Absolute quantification and relative quantification were performed using the obtained Ct value.

In the present invention, the structure of the amplicon, which is the design nucleic acid, is the same as that shown in [General Formula (I)], and the amplicon is amplified using the same PCR primer pair as shown in [General Formula (I)]. Accordingly, the amplification efficiencies of the design nucleic acids representing the target biomolecule and the standard material for quality control and measurement may be the same.

In the present invention, the amount of the design nucleic acid of the complex formed by the target biomolecule in the sample to be analyzed, to which the standard material for quality control and measurement was artificially added, was analyzed by the Ct (cycle threshold) value obtained in the above-described Example using a $2^{-Ct}$ method (comparative Ct method) (Livak KJ. Comparative Ct Method. ABI PRISM 7700 Sequence Detection System. User Bulletin No.2. Relative Quantitation of Gene Expression. PE Applied Biosystems, 1997.).

In this Ct method, quantification may be performed without preparing a calibration curve. However, it is presupposed that the PCR amplification efficiency is almost constant for all biomolecules to be measured.

The analyzed Ct value of the design nucleic acid of the complex of the target biomolecule in the sample to be analyzed, to which the standard material is added, is normalized using the Ct value of the design nucleic acid of the standard material complex ($Ct_{target\ biomolecule}$ - $Ct_{external}$ standard material) to thus secure $\Delta Ct$-n, and then the concentration of the target biomolecule in the sample to be analyzed, to which the standard material is added, is calculated as follows:

Concentration of external standard material x $2^{-\Delta Ct-n}$

When the binding affinity between the external standard material and the specific ligand thereof is different from the binding affinity between the target biomolecule and the specific ligand thereof, the decided concentration of the target biomolecule may not accurately reflect the concentration of the target biomolecule in the sample. Therefore, it is preferable to perform calibration with respect to the binding affinity difference.

In order to perform this calibration, the target biomolecules and the target external standard material are quantified, and one or more control samples containing the target biomolecules and the target external standard material at the same concentration are prepared. The control sample(s) are used to obtain the Ct value (or the average of the values when the number of control samples is two or more) calculated from the amplified substance of the design nucleic acid of the complex of the target biomolecule and the Ct value (or the average of the values when the number of control samples is two or more) calculated from the amplified substance of the design nucleic acid of the complex of the external standard material, thus generating ACt-c, which is the difference between the Ct values ($Ct_{target\ biomolecule\ of\ control\ sample}$ - $Ct_{external\ standard\ material\ of\ control\ sample}$). Next, ACt-cal ($\Delta Ct$-n - $\Delta Ct$-c) may be obtained by performing calibration of $\Delta Ct$-n using the difference value, and the concentration of the target biomolecules may be obtained from the concentration of the external standard material according to the following equation using ACt-cal, thereby securing the result that reflects the difference between the binding affinity.

Concentration of external standard material x $2^{-\Delta Ct-cal}$

Therefore, it can be said that the target biomolecules in the sample to be analyzed are present at a concentration which is $2^{-\Delta Ct-cal}$ times higher than the concentration of the external standard material.

Example 8. Analysis of biomolecules using length of design nucleic acid

One or more target biomolecules in the sample, to which the standard material for quality control and measurement was added, were analyzed using the length of the designed nucleic acid according to capillary electrophoresis for analyzing the length of the design nucleic acid.

In the above-described Example, in both cases where the standard material complexes for quality control and measurement formed in one tube and the eight types of target biomolecule complexes were harvested using a magnet or where the complexes were harvested using an ELISA method, the PCR was performed to secure amplified products.

A mixture solution, in which a 20 μM Cy5-labeled universal PCR forward primer (5'-Cy5-CGGAAGCGTGCTGGGCC-3) and a 10 μM universal PCR backward primer (5'-TCGACCTCTGGGTTATG-3') were added to the harvested complex and which had a reaction volume adjusted to 20 μl using tertiary distilled water, was left in pfu-PCR premix (Bioneer, Korea) at 95°C for 30 seconds, at 60°C for 30 seconds, and at 72°C for 30 seconds, and this was repeated 35 times, thus amplifying a complete double-stranded nucleic acid.

In order to analyze the amplified product of the complex design nucleic acid using the length of the nucleic acid, the amplified product was subjected to capillary electrophoresis. Capillary electrophoresis was performed using an ABI 3130XL Genetic Analyzer (36-cm capillary array and POP7 polymer; Applied Biosystems, USA) according to the protocol provided. 1.0 μl of the PCR reactant was mixed with 9 L of Hi-Di formamide, reacted at 80°C for 2 minutes, and placed on ice. Samples were injected into a capillary tube and a voltage of 1.6 kV was applied thereto for 15 seconds, followed by capillary electrophoresis at an electrophoresis voltage of 10 kV and a temperature of 60°C.

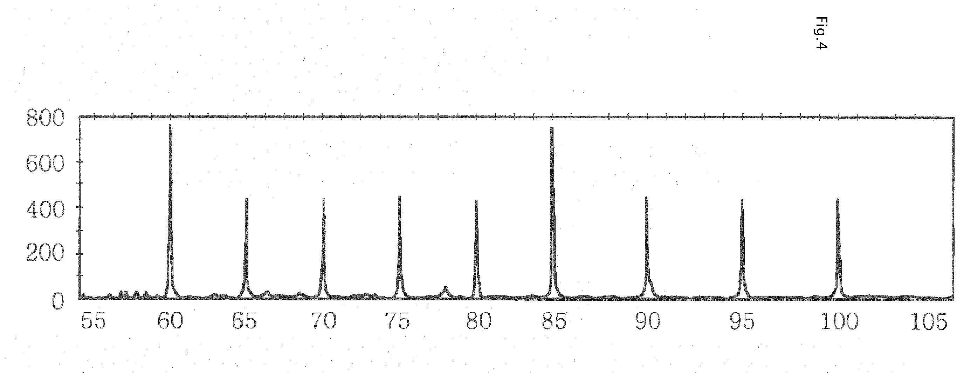

FIG. 4 is a view showing the result of analysis of the amplified product obtained by performing PCR of the design nucleic acids of the complexes using capillary electrophoresis in both cases where the complexes of eight types of biomolecules in the Example of the present invention and the standard material for quality control and measurement are harvested using a magnet and where they are harvested using an ELISA method.

From the result of analysis of the amplified product that was obtained by performing the PCR of the design nucleic acids of the formed standard material complexes using capillary electrophoresis in both cases where these complexes formed during analysis of 8 types of target biomolecules in the sample containing the standard material for quality control and measurement in one tube were harvested using a magnet and where the complexes were harvested using an ELISA method, it can be seen that the standard materials for quality control and measurement and the target biomolecules were preferably analyzed in the sample to be analyzed using the ligand PCR according to the present invention.

Further, in both cases where the formed complexes were harvested using the magnet and the ELISA method, after the results of capillary electrophoresis of the amplified product that was obtained through PCR of the design nucleic acids of the harvested standard material and target biomolecule complexes were secured, the result of analysis of the target biomolecules and the result of analysis of the standard material could be compared and analyzed, thus ultimately quantifying the target biomolecules or calculating the quantitative ratio of the standard material/target biomolecule or the quantitative ratio of the target biomolecules.

In the result (electropherogram) of FIG. 4 of capillary electrophoresis, each peak means a corresponding amplified substance according to the length thereof, and the area thereof means the amount of the amplified substance. Accordingly, since the external standard material is used at a constant concentration, the peak area of the target biomolecule and the peak area of the external standard material may be compared and calculated, thereby obtaining the concentration of the target biomolecules in the sample. Further, as in the Ct method, capillary electrophoresis may be performed using the control sample containing the target biomolecule and the external standard material at the same concentration, thus reflecting the difference in binding affinity with the ligands. When the binding affinity difference is reflected, the concentration of the target biomolecules in the sample may be calculated more accurately,

Example 9. Analysis of biological meaning

Blood, which is a biosample, is used to examine chronic diseases such as acute myocardial infarction or sepsis. In particular, three to four typical biomolecules for patient monitoring are examined, and only the cut-off value of the result is used for diagnosis.

An *in-vitro* diagnostic multivariate index assay (IVDMIA), which is a method to quantitatively analyze and to diagnose the functional relation between a normal group and a patient group based on the results of the quantitative test of various target biomolecules, includes an apparatus or a technique for producing classification, points, and indexes as the specific result of a patient by combining the multiple results of the target biomolecules using analytical functions in order to diagnose, cure, mitigate, treat, and prevent diseases or other conditions that are biologically meaningful.

In a clinical decision support system for determining biological meaning of the present invention, data obtained by calibrating analysis results of the amplified products of the design nucleic acids in the standard material, the target biomolecules, and the ligand complexes, which are generated using an apparatus for analyzing amplified products (a real-time PCR which is an apparatus for checking the base sequence of a nucleic acid or a capillary electrophoresis apparatus which is an apparatus for checking the length of a nucleic acid), and patient's information from an OCS (operation control system) are used as inputs. The output has a screen for inputting and checking the analysis results by a clinician, and a screen for visually explaining the risk and classification to the patient by a diagnostician. A biological meaning analysis system must have an amplified product analyzer, OCSs, and interfaces, and the results obtained using data mining must be stored in DBs. The system also has an XML repository to handle the XML data defined in HL7. For data mining, necessary items may be set up and mining engines may be controlled.

As shown in FIG. 4, the learning engine module of the clinical decision support system is an artificial intelligence learning engine module required for determining a disease using the result of analysis of the amplified product of the design nucleic acid representing the target biomolecules.

The biological meaning analysis system supports HL7. When data are processed within the biological meaning analysis system, a data structure is designed so as to include the Schema defined in HL7. The system has an XML repository module and an XML repository client in order to store and use Schema information of XML documents.

The system includes an amplified product analysis result interface module for the purpose of linking with an amplified product analyzer for analyzing the amplified product of the design nucleic acid representing the target biomolecules. In the case of providing a SDK (software development kit) based on the result of analysis of the amplified product, a gateway is basically developed. The OCS interface module reads the patient's information from the OCS and provides the information needed for data mining. Further, the disease information analyzed using data mining is stored in the OCS (LIS).

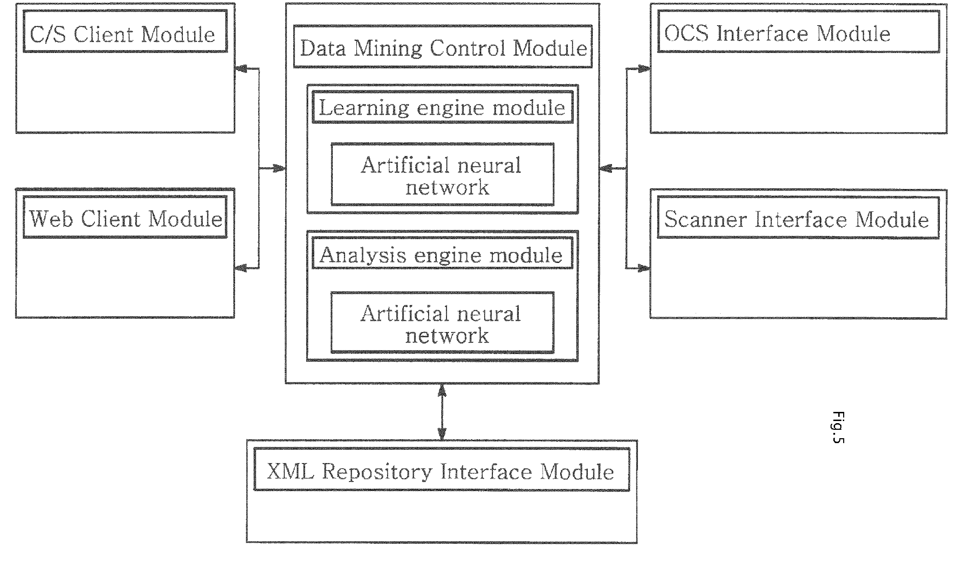

As shown in FIG. 5, the biological meaning analysis of the clinical decision support system broadly includes four partial systems. In the clinical decision support system, a data analysis/prediction model generation module and a diagnostic client module, which are a biological meaning determination engine, an amplified product analyzer interface, which is a linking module with an amplified product analyzer for generating aptamer machine data, and a communication server for communication between different modules and data storage are organically combined. The operation of the system is broadly divided into a system building step and a system application step.

In the system building step, a set of normal/patient samples configured to reflect the characteristics of the disease is constructed, and the target biomolecules are subjected to ligand-nucleic-acid analysis using the standard material for quality control and measurement with respect to the corresponding set of samples. Information on the design nucleic acids of the target biomolecule-ligand complexes and the standard material ligand complexes is input into the system using the amplified product analyzer interface module, and is basically pre-processed using the data analysis/prediction model generation module, which generates learning data of an artificial neural network algorithm and also a biological meaning analysis model using the learning data based on the artificial neural network algorithm. The biological meaning analysis model that is generated is stored in a computer.

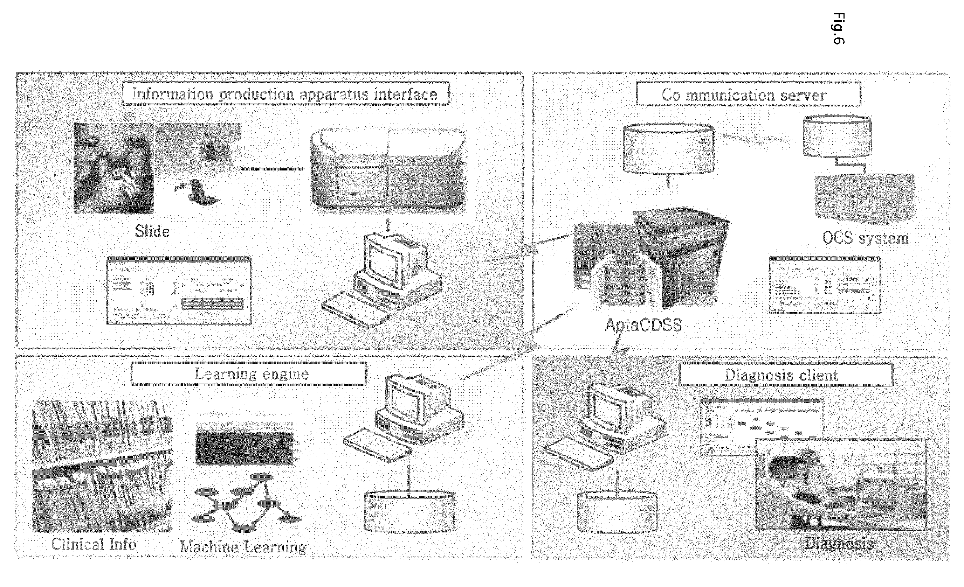

As shown in FIG. 6, the system application step includes mounting the biological meaning analysis model stored in the DB on a biological meaning analysis client, operating the biological meaning analysis client, performing the ligand-nucleic-acid analysis of a biological meaning analysis subject, generating information on the result of analysis using the amplified product analyzer, inputting data using the amplified product analyzer interface, visualizing the analysis and results of the biological meaning in the biological meaning analysis client, and storing the result of the biological meaning analysis in the DB.

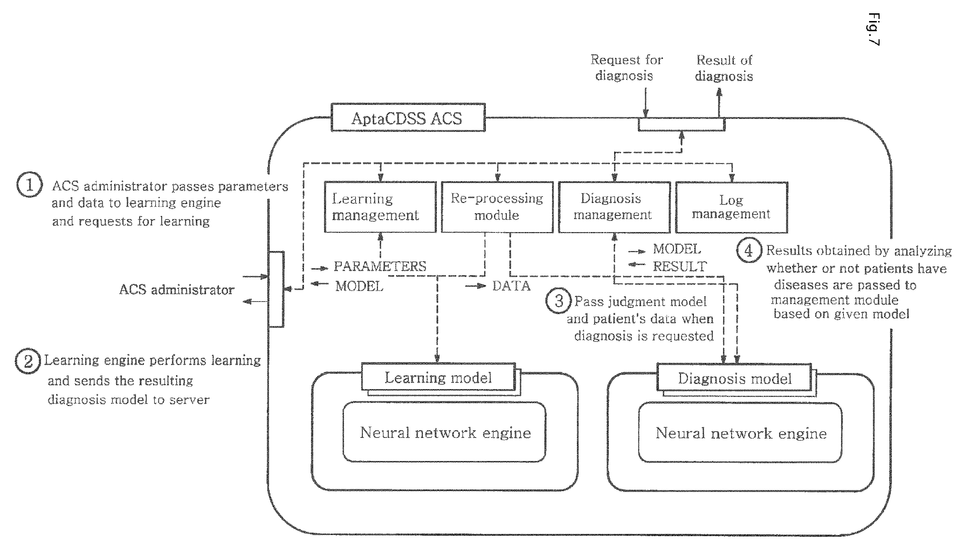

As shown in FIG. 7, the input data of the system include clinical information such as blood sample target biomolecule information, personal information, and results of other medical treatments performed on a patient. The ligand-nucleic-acid analysis method is an analytical technique for measuring the result of analysis of a design nucleic acid in a target biomolecule-ligand complex using a design nucleic acid of a standard material-ligand complex in a sample. The distribution pattern of target biomolecules related to diseases may be grasped from the blood sample of a patient using the ligand-nucleic-acid analysis method. With respect to normal and patient samples, only information obtained from the same target biomolecule in all experiments is used for learning and testing. Information on the result of analysis of the target biomolecule set is primarily obtained by analyzing the amplified product of the design nucleic acid that represents the target biomolecule, and is digitized via a Ct (cycle threshold) analysis step of the amplified product using real-time PCR, which is an amplified product analyzer.

As shown in FIG. 7, each Ct value of the analysis result data of the target biomolecules indicates the extent of the target biomolecule pattern. The numerical data are obtained from the Ct data of the amplified product, each value thereof may mean the value of one target biomolecule, and the numerical value thereof may mean the extent of the target biomolecule pattern.

In addition to the target biomolecule information, clinical information on each patient is input from the system. Clinical information about each patient, such as the age, gender, blood pressure, history of disease in family, and smoking status, is also input. All of these data are stored in the database. The clinical data used for building the system are received from collaborating medical institutions. Experimental data of the design nucleic acid analysis of the target biomolecule-ligand complex are used as the target biomolecule pattern data.

The Ct data that are input are used as Ct value information corresponding to the target biomolecules in the ligand-nucleic-acid analysis method of one or more target biomolecules.

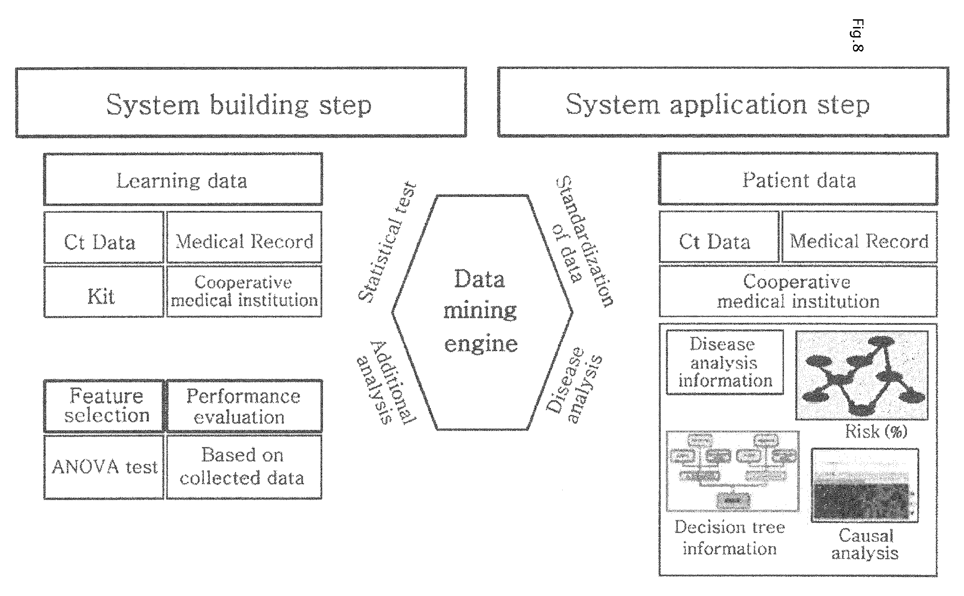

As shown in FIG. 8, the experimental results of the ligand-nucleic-acid analysis are converted to numerical data using an amplified product analyzer. In order to directly use these data in disease biological meaning determination systems, it is necessary to link the biological meaning determination system with an amplified product analyzer capable of reading the Ct data of the amplified product of the design nucleic acid that represents the target biomolecules. Therefore, based on the use of an amplified product analyzer that provides an SDK, a gateway for linking with a clinical decision support system for determining a biological meaning is developed.

Meanwhile, patient information from the OCS system is input as clinical data for biological meaning determination. The patient information from the OCS is read using an OCS interface module, and the information necessary for data mining is provided to a system engine.

As shown in FIG. 8, indices for biological meaning determination of disease are predicted based on information in the database. By applying various machine learning methods using stored information, it is possible to analyze the indexes for diseases. Representative machine-learning methods such as an artificial neural network, a decision tree, a Bayesian network, and a support vector machine (SVM) are used as key algorithms for analysis. In general, these methods allow the prediction of new data by learning the vector form of numerical data provided as an input. Having excellent performance in classification of data, the artificial neural network and the support vector machine are the most popular classification algorithms in the study on the machine learning field. Further, the decision tree and the Bayesian network have merit in that the result is visualized so as to be easily understood. In this biological meaning determination system, the learning model is optimized via continuous testing using the artificial neural network algorithm, which realizes excellent prediction performance of the disease indices.

When information on the Ct analysis result produced using a ligand-nucleic-acid analysis method of one or more target biomolecules for a new patient using a standard material for quality control and measurement and clinical information are input, the analysis system shows the results of analysis including indexes for specific diseases in various forms. The analysis system shows information that helps doctors make decisions about the disease. The client may show simple numerical information on disease indexes and also visualized disease indexes using biological networks. The results of analysis on specific diseases may help not only medical staff but also patients to more easily understand the indices related to their diseases and their causes.

The communication server controls communication by the clinical decision support system that determines the biological meaning. The communication server controls all parts related to communication of the data, including an amplified product analyzer interface, a biological meaning determination engine, a biological meaning determination client, and a database server. That is, the patient's experimental information is input to the DB, and the biological meaning determination engine fetches and diagnoses the patient's experimental information, stores the biological meaning determination result in the DB, and outputs the biological meaning determination result to a biological meaning determination client screen, which is responsible for all aspects of data management. In addition, the database server stores various data such as patient's test information, patient's experimental information, learning information required for determining the biological meaning, biological meaning determination engine model information, system setting information, system log information, and biological meaning determination result information. The communication server and the database have a close relationship with each other and manage data transmission for each module.

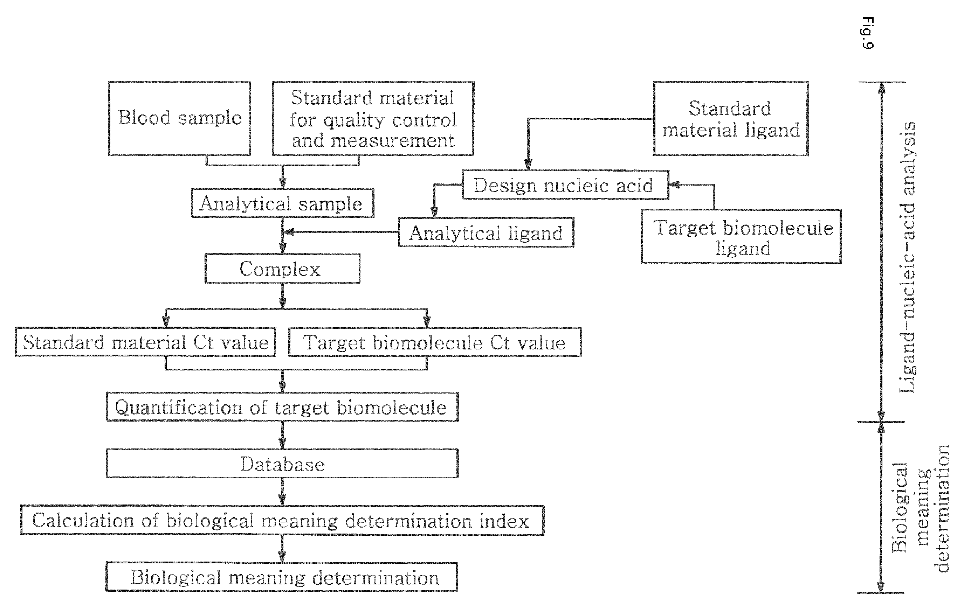

As shown in FIG. 9, the blood sample to which the standard material for quality control and measurement is added is analyzed using the ligand-nucleic-acid analysis method, and on the basis of the result of analysis of the standard material in the blood sample, the result of analysis of the target biomolecules may be checked. Further, the biological meaning may be decided by confirming that the results of analysis of serum biomolecules in acute myocardial infarction patients and sepsis patients are different.

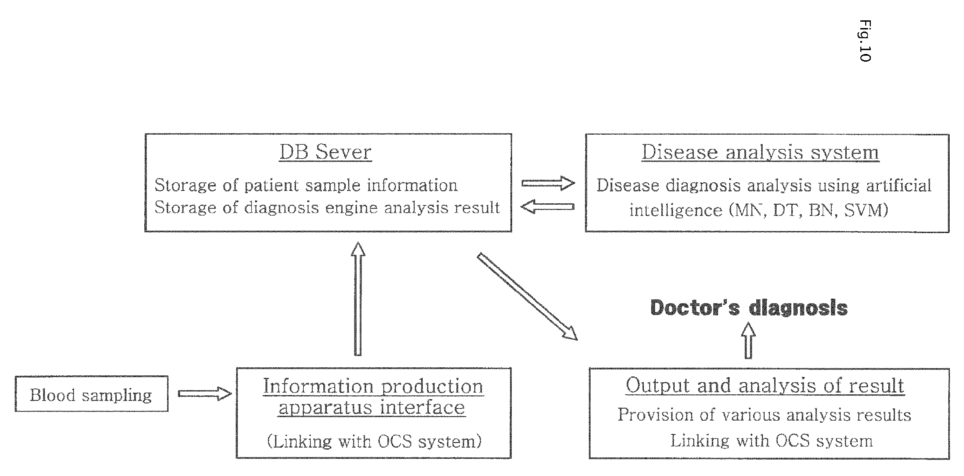

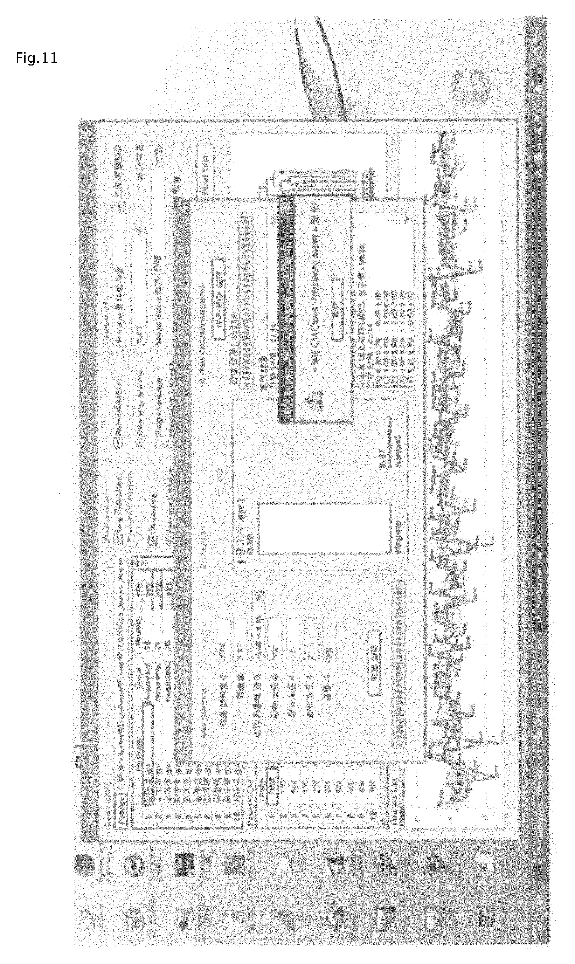

FIG. 10 is a flowchart for diagnosing diseases by producing the result of analysis of biomolecules of a blood sample of a specific person using a clinical decision support system including a database, which includes the produced analysis results constituted for each disease group, and an artificial neural network. As shown in FIG. 11, the biological meaning may be decided using useful information obtained by analyzing a database including the analysis results produced in many biosamples using a bioinformatics technique.

While the present invention has been particularly shown and described with reference to specific embodiments thereof, those skilled in the art will appreciate that this specific description is merely an example, and the scope of the present invention is not limited thereto. Accordingly, the actual scope of the present invention will be defined by the appended claims and their equivalents.

### Industrial Applicability

Target biomolecules in a sample may be analyzed using a ligand-nucleic-acid analysis method of the present invention in a single test, thereby checking the biological meaning of the target biomolecules in the sample, and highly sensitive detection is obtained using PCR and amplification of the fluorescent signal indicating the presence thereof. Therefore, the present invention provides a method of efficiently determining disease screening, disease diagnosis, sensitivity to diseases, and a difference between individuals, such as a difference in response to therapeutic agents for each sample, by analyzing the target biomolecules in a single test together with internal quality control, thereby contributing greatly to overcoming human disease and providing a very useful effect in the medical industry on the economic level.

### Claims

1.    A method of analyzing biomolecules using external biomolecules as a standard material, the method comprising:

(a) preparing a biosample containing one or more target biomolecules or a processed sample thereof;
(b) quantifying the one or more external biomolecules which are not present in the biosample or the processed sample thereof as the standard material, and adding the external biomolecules;
(b) adding a connection structure of a ligand and a design nucleic acid specifically binding to the target biomolecules or the external biomolecules to thus form complexes of the target biomolecules or the external biomolecules and the specific connection structure thereof;
(c) separating the complexes;
(d) amplifying the design nucleic acids of the complexes; and

(e) obtaining quantitative information on the target biomolecules by quantifying an amplified substance of the design nucleic acid of the complex of the target biomolecules and an amplified substance of the design nucleic acid of the complex of the external biomolecules, followed by comparison,

wherein the design nucleic acid includes a region binding to a forward primer, a region binding to a backward primer, and between these regions, a region representing molecules specifically binding to the ligand so that the molecules are capable of being identified, and
the region binding to the forward primer and the region binding to the backward primer have a common base sequence in all of the design nucleic acids so that all of the design nucleic acids are amplified using one set of the forward primer and the backward primer.

2. The method of claim 1, wherein the obtaining the quantitative information on the target biomolecules in step (e) includes reflecting a difference between a binding affinity of the target biomolecules and the connection structure thereof and a binding affinity of the external biomolecules and the connection structure thereof.

3. The method of claim 2, wherein the reflecting includes:

(i) quantifying the target biomolecules and the external biomolecules to thus prepare a control sample including the target biomolecules and the external biomolecules at a same concentration;
(ii) adding the connection structure which is specifically binding to the quantified target biomolecules or the quantified external biomolecules of the control sample and which is identical with the connection structure used in step (c) to thus form a complex of the target biomolecules and the specific connection structure thereof and a complex of the external biomolecules and the specific connection structure thereof;
(iii) separating the complexes;
(iv) amplifying design nucleic acids of the complexes;
(v) obtaining a difference between an amplified substance for the target biomolecules and an amplified substance for the external biomolecules; and
(vi) reflecting the difference to the quantitative information on the target biomolecules in step (e).

4. The method of claim 3, wherein all amplifications are performed according to a real-time polymerase chain reaction for detecting the amplified substance in real time, an amount of the amplified substance is calculated using a Ct (threshold cycle) value, a result of quantification and comparison of step (e) is calculated using $\Delta$Ct-n ($Ct_{\text{target biomolecule}} - Ct_{\text{external biomolecule}}$), the quantitative information on the target biomolecules in step (e) is calculated using a concentration of the external biomolecules in step (a) $\times$ $2^{-\Delta Ct\text{-}n}$, and the difference in step (v) is calculated using $\Delta$Ct-c ($Ct_{\text{biomolecule of control sample}} - Ct_{\text{external biomolecule of control sample}}$), such that $\Delta$Ct-cal ($\Delta$Ct-n - $\Delta$Ct-c), which is a differential value of $\Delta$Ct-n and $\Delta$Ct-c, is reflected to step (vi), thus deciding the quantitative information on the target biomolecules in step (e) as the concentration of the external biomolecules in step (a) $\times$ $2^{-\Delta Ct\text{-}cal}$.

5. The method of claim 1, wherein two or more types of the target biomolecules are present in the sample, and thus, two or more types of the connection structures of the ligand and the design nucleic acid specific to the biomolecules are used, and, in the design nucleic acids of the two or more types of the connection structures, the region binding to the forward primer and the region binding to the backward primer have the common base sequence in the design nucleic acids of all of the connection structures regardless of the type of the connection structures.

6. The method of claim 1, wherein the biomolecules are a protein or a glycoprotein, and the ligand is an antibody.

7. The method of claim 1, wherein the design nucleic acid is a single-stranded DNA or a single-stranded RNA, and the region representing the molecules specifically binding to the ligand is a region representing the molecules by the base sequence or a length thereof.

8. The method of claim 1, wherein an amplification technique includes any one polymerase chain reaction among a polymerase chain reaction (PCR), a real-time polymerase chain reaction (real-time PCR), a reverse-transcription-polymerase chain reaction (RT-PCR), a real-time reverse-transcription-polymerase chain reaction (real-time RT-PCR), a multiplex polymerase chain reaction (multiplex PCR), a real-time multiplex polymerase chain reaction (real-time multiplex PCR), and a multiplex reverse-transcription-polymerase chain reaction (multiplex RT-PCR).

9. The method of claim 1, wherein the amplifying in step (c) includes a polymerase chain reaction selected from the group consisting of a real-time polymerase chain reaction (real-time PCR), a real-time reverse-transcription-polymerase chain reaction (real-time RT-PCR), a real-time multiplex polymerase chain reaction (real-time multiplex PCR), and a real-time multiplex reverse-transcription-polymerase chain reaction (real-time multiplex RT-PCR), and a Taqman probe is added during the amplifying to quantify the amplified substance.

**10.** A kit for analyzing biomolecules using external biomolecules as a standard material, the kit comprising:

one or more types of connection structures of a ligand and a design nucleic acid specifically binding to each of one or more types of target biomolecules; and
one or more types of connection structures of the ligand and the design nucleic acid specifically binding to each of one or more types of the external biomolecules, which are not present in a biosample containing the target biomolecules, as the standard material,
wherein the design nucleic acid includes a region binding to a forward primer, a region binding to a backward primer, and between these regions, a region representing molecules specifically binding to the ligand so that the molecules are capable of being identified, and
the region binding to the forward primer and the region binding to the backward primer have a common base sequence in all of the design nucleic acids so that all of the design nucleic acids are amplified using one set of the forward primer and the backward primer.

**11.** The kit of claim 10, further comprising:

one or more types of target biomolecules and one or more types of external biomolecules to be used in a control sample in order to determine a difference between a binding affinity of the target biomolecules and the connection structures thereof and a binding affinity of the external biomolecules that are the standard material and the connection structures thereof.

**12.** The kit of claim 10, further comprising:

an instruction manual of the kit,

wherein the instruction manual teaches a method including following steps (a) to (e) as a method of analyzing the biomolecules:

(a) preparing the biosample containing the one or more target biomolecules or a processed sample thereof;
(b) quantifying the external biomolecules which are not present in the biosample or the processed sample thereof as the standard material, and adding the external biomolecules;
(b) adding a connection structure of the ligand and the design nucleic acid specifically binding to the target biomolecules or the external biomolecules to thus form complexes of the target biomolecules or the external biomolecules and the specific connection structure thereof;
(c) separating the complexes;
(d) amplifying the design nucleic acids of the complexes; and
(e) obtaining quantitative information on the target biomolecules by quantifying an amplified substance of the design nucleic acid of the complex of the target biomolecules and an amplified substance of the design nucleic acid of the complex of the external biomolecules, followed by comparison.

**13.** The kit of claim 12, wherein the manual further teaches that the obtaining the quantitative information on the target biomolecules in step (e) includes reflecting a difference between a binding affinity of the target biomolecules and the connection structure thereof and a binding affinity of the external biomolecules and the connection structure thereof.

**14.** The kit of claim 13, wherein the manual further teaches that the reflecting includes:

(i) quantifying the target biomolecules and the external biomolecules to thus prepare a control sample including the target biomolecules and the external biomolecules at a same concentration;
(ii) adding the connection structure which is specifically binding to the quantified target biomolecules or the quantified external biomolecules of the control sample and which is identical with the connection structure used in step (c) to thus form a complex of the target biomolecules and the specific connection structure thereof and a complex of the external biomolecules and the specific connection structure thereof;
(iii) separating the complexes;

(iv) amplifying design nucleic acids of the complexes;

(v) obtaining a difference between an amplified substance for the target biomolecules and an amplified substance for the external biomolecules; and

(vi) reflecting the difference to the quantitative information on the target biomolecules in step (e).

15. The kit of claim 14, wherein the manual further instructs that all amplifications are performed according to a real-time polymerase chain reaction for detecting the amplified substance in real time, an amount of the amplified substance is calculated using a Ct (threshold cycle) value, a result of quantification and comparison of step (e) is calculated using $\Delta$Ct-n (Ct$_{target\ biomolecule}$ - Ct$_{external\ biomolecule}$), the quantitative information on the target biomolecules in step (e) is calculated using a concentration of the external biomolecules in step (a) $\times$ $2^{-\Delta Ct\text{-}n}$, and the difference in step (v) is calculated using $\Delta$Ct-c (Ct$_{biomolecule\ of\ control\ sample}$ - Ct$_{exrernal\ biomolecule\ of\ control\ sample}$), such that $\Delta$Ct-d ($\Delta$Ct-n - $\Delta$Ct-c), which is a differential value of $\Delta$Ct-n and $\Delta$Ct-c, is reflected to step (vi), thus determining the quantitative information on the target biomolecules in step (e) as the concentration of the external biomolecules in step (a) $\times$ $2^{-\Delta Ct\text{-}d}$.

16. The kit of claim 10, wherein two or more types of the target biomolecules are analyzed using the kit.

17. The kit of claim 10, wherein the biomolecules are a protein or a glycoprotein, and the ligand is an antibody.

18. The kit of claim 10, wherein the design nucleic acid is a single-stranded DNA or a single-stranded RNA, and the region representing the molecules specifically bonded to the ligand is a region representing the molecules by the base sequence or a length thereof.

19. The kit of claim 12, wherein the manual further teaches that the amplifying includes any one polymerase chain reaction among a polymerase chain reaction (PCR), a real-time polymerase chain reaction (real-time PCR), a reverse-transcription-polymerase chain reaction (RT-PCR), a real-time reverse-transcription-polymerase chain reaction (real-time RT-PCR), a multiplex polymerase chain reaction (multiplex PCR), a real-time multiplex polymerase chain reaction (real-time multiplex PCR), and a multiplex reverse-transcription-polymerase chain reaction (multiplex RT-PCR).

20. The kit of claim 12, wherein the manual further teaches that the amplifying includes a polymerase chain reaction selected from the group consisting of a real-time polymerase chain reaction (real-time PCR), a real-time reverse-transcription-poiymerase chain reaction (real-time RT-PCR), a real-time multiplex polymerase chain reaction (real-time multiplex PCR), and a real-time multiplex reverse-transcription-polymerase chain reaction (real-time multiplex RT-PCR), and that a Taqman probe is added during the amplifying to quantify the amplified substance.

Fig.1

Fig.2

Fig.3

Fig.4

Fig.5

OCS Interface Module

Scanner Interface Module

Data Mining Control Module

Learning engine module

Artificial neural network

Analysis engine module

Artificial neural network

XML Repository Interface Module

C/S Client Module

Web Client Module

Fig.6

Fig.7

AptaCDSS ACS

Request for diagnosis

Result of diagnosis

Learning management

Re-processing module

Diagnosis management

Log management

PARAMETERS

MODEL

DATA

MODEL

RESULT

Learning model

Neural network engine

Diagnosis model

Neural network engine

① ACS administrator passes parameters and data to learning engine and requests for learning

ACS administrator

② Learning engine performs learning and sends the resulting diagnosis model to server

③ Pass judgment model and patient's data when diagnosis is requested

④ Results obtained by analyzing whether or not patients have diseases are passed to management module based on given model

Fig.8

Fig.9

Ligand-nucleic-acid analysis

Biological meaning determination

Standard material ligand

Target biomolecule ligand

Design nucleic acid

Analytical ligand

Standard material for quality control and measurement

Blood sample

Analytical sample

Complex

Target biomolecule Ct value

Standard material Ct value

Quantification of target biomolecule

Database

Calculation of biological meaning determination index

Biological meaning determination

Fig.10

EP 3 287 530 A1

**DB Sever**

Storage of patient sample information
Storage of diagnosis engine analysis result

**Disease analysis system**

Disease diagnosis analysis using artificial
intelligence (MN, DT, BN, SVM)

Blood sampling

**Information production
apparatus interface**

(Linking with OCS system)

**Doctor's diagnosis**

**Output and analysis of result**

Provision of various analysis results
Linking with OCS system

Fig.11

| INTERNATIONAL SEARCH REPORT | International application No. |
|---|---|
| | **PCT/KR2016/004130** |

**A. CLASSIFICATION OF SUBJECT MATTER**

*C12Q 1/68(2006.01)i, B01L 7/00(2006.01)i, G06F 19/00(2011.01)i*

According to International Patent Classification (IPC) or to both national classification and IPC

**B. FIELDS SEARCHED**

Minimum documentation searched (classification system followed by classification symbols)
C12Q 1/68; C12M 1/00; C12N 15/10; G06F 19/10; C12N 15/11; B01L 7/00; G06F 19/00

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)

eKOMPASS (KIPO internal) & Keywords: in vitro biomolecule, target biomolecule, fixed quantity, ligand, amplification, nucleic acid design, universal primer

**C. DOCUMENTS CONSIDERED TO BE RELEVANT**

| Category* | Citation of document, with indication, where appropriate, of the relevant passages | Relevant to claim No. |
|---|---|---|
| A | KR 10-2014-0118680 A (KIM, Sung Chun) 08 October 2014<br>See abstract; and claims 1-35. | 1-20 |
| A | KR 10-2010-0058566 A (FLUIDIGM CORPORATION) 03 June 2010<br>See abstract; and claims 1-17. | 1-20 |
| A | JP 2012-135306 A (F HOFFMANN LA ROCHE AG.) 19 July 2012<br>See the entire document. | 1-20 |
| A | KR 10-2014-0129644 A (GENEMATRIX INC.) 07 November 2014<br>See the entire document. | 1-20 |
| A | KR 10-2013-0007961 A (SAMSUNG ELECTRONICS CO., LTD.) 21 January 2013<br>See the entire document. | 1-20 |

| ☐ Further documents are listed in the continuation of Box C. | ☒ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 09 AUGUST 2016 (09.08.2016) | **09 AUGUST 2016 (09.08.2016)** |

| Name and mailing address of the ISA/KR<br>Korean Intellectual Property Office<br>Government Complex-Daejeon, 189 Seonsa-ro, Daejeon 302-701,<br>Republic of Korea<br>Facsimile No. 82-42-472-7140 | Authorized officer<br><br>Telephone No. |
|---|---|

Form PCT/ISA/210 (second sheet) (January 2015)

# EP 3 287 530 A1

**INTERNATIONAL SEARCH REPORT**
Information on patent family members

International application No.

**PCT/KR2016/004130**

| Patent document cited in search report | Publication date | Patent family member | Publication date |
|---|---|---|---|
| KR 10-2014-0118680 A | 08/10/2014 | WO 2015-060511 A1 | 30/04/2015 |
| KR 10-2010-0058566 A | 03/06/2010 | AU 2008-295992 A1 | 12/03/2009 |
| | | AU 2008-295992 B2 | 17/04/2014 |
| | | CA 2698545 A1 | 12/03/2009 |
| | | CA 2698545 C | 08/07/2014 |
| | | CN 101821619 A | 01/09/2010 |
| | | CN 101821619 B | 11/02/2015 |
| | | EP 2198293 A1 | 23/06/2010 |
| | | EP 2198293 B1 | 18/01/2012 |
| | | JP 2010-538614 A | 16/12/2010 |
| | | JP 5707132 B2 | 22/04/2015 |
| | | WO 2009-033178 A1 | 12/03/2009 |
| JP 2012-135306 A | 19/07/2012 | CA 2761546 A1 | 17/06/2012 |
| | | CN 102559875 A | 11/07/2012 |
| | | EP 2465945 A1 | 20/06/2012 |
| | | JP 5871600 B2 | 01/03/2016 |
| | | US 2012-0190010 A1 | 26/07/2012 |
| KR 10-2014-0129644 A | 07/11/2014 | WO 2014-178562 A1 | 06/11/2014 |
| KR 10-2013-0007961 A | 21/01/2013 | CN 102876660 A | 16/01/2013 |
| | | EP 2546361 A1 | 16/01/2013 |
| | | EP 2546361 B1 | 03/06/2015 |
| | | US 2013-0017549 A1 | 17/01/2013 |
| | | US 8945844 B2 | 03/02/2015 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4683195 A
- US 4683202 A
- US 4800159 A
- WO 9001069 A
- EP 439182 A
- US 6410276 B
- US 4437975 A
- US 5413909 A
- US 5861245 A
- US 5130238 A
- US 5409818 A

- US 5554517 A
- US 6063603 A
- US 5925517 A
- US 5210015 A
- US 5487972 A
- US 5763181 A
- US 3996345 A
- US 4351760 A
- US 6369893 B
- US 6391541 B

**Non-patent literature cited in the description**

- **SMITH et al.** *Mol Cell Protomics,* 18 November 2003
- **MCCAULEY et al.** *Anal Biochem,* 2003, vol. 319 (2), 244-250
- **ADAM et al.** *Cancer Research,* 2002, vol. 62, 3609-3614
- **LI et al.** *Clinical Chemistry,* 2002, vol. 48, 1296-1304
- **PETRICOIN et al.** *The Lancet,* 2002, vol. 359, 572-577
- **SANO et al.** *Science,* 1992, vol. 258, 120-122
- Molecular Cloning. **SAMBROOK et al.** A Laboratory Manual. Cold Spring Harbor Press, 2001
- **STEMMER, W.P. et al.** *Gene,* 1995, vol. 164, 49-53
- **CARLSON, B.** *Genet Eng Biotechn N,* 2008, vol. 28, 12-12
- **SANTALUCIA, J.** *Proc Natl Acad Sci U S A,* 1998, vol. 95, 1460-1465
- FLUORESCENCE SPECTROSCOPY. Marcel Dekker, 1971
- **WHITE et al.** FLUORESCENCE ANALYSIS: A PRACTICAL APPROACH. Marcel Dekker, 1970
- **BERLMAN.** HANDBOOK OF FLUORESCENCE SPECTRA OF AROMATIC MOLECULES. Academic Press, 1971

- **GRIFFITHS.** COLOUR AND CONSTITUTION OF ORGANIC MOLECULES. Academic Press, 1976
- INDICATORS. Pergamon Press, 1972
- Molecular Probes. **HAUGLAND.** HANDBOOK OF FLUORESCENT PROBES AND RESEARCH CHEMICALS. 1992
- **PRINGSHEIM.** FLUORESCENCE AND PHOSPHO-RESCENCE. Interscience Publishers, 1949
- **HAUGLAND, R.P.** Molecular Probes. *HANDBOOK OF FLUORESCENT PROBES AND RESEARCH CHEMICALS,* 1996
- **LEE, D.H. TOCE.** *Innovative Technology for High Multiplex Real-time PCR. Seegene bulletin,* 2012, vol. 1, 510
- **LIVAK KJ.** Comparative Ct Method. ABI PRISM 7700 Sequence Detection System. User Bulletin No. 2. Relative Quantitation of Gene Expression. *PE Applied Biosystems,* 1997
- **INNIS et al.** *PCR Protocols: A Guide to Methods and Applications,* 1990
- **LIVAK KJ.** Comparative Ct Method. ABI PRISM 7700 Sequence Detection System. User Bulletin No.2. Relative Quantitation of Gene Expression. *PE Applied Biosystems,* 1997